(19) Europäisches Patentamt / European Patent Office / Office européen des brevets

(11) EP 4 434 528 A1

(12) EUROPEAN PATENT APPLICATION
published in accordance with Art. 153(4) EPC

(43) Date of publication:
25.09.2024 Bulletin 2024/39

(51) International Patent Classification (IPC):
A61K 31/7088 (2006.01)    C12N 15/62 (2006.01)
A61P 35/00 (2006.01)

(21) Application number: 23799306.8

(22) Date of filing: 06.05.2023

(86) International application number:
PCT/CN2023/092440

(87) International publication number:
WO 2023/213320 (09.11.2023 Gazette 2023/45)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 06.05.2022   CN 202210486911
04.05.2023   CN 202310493868

(71) Applicant: Liverna Therapeutics Inc.
Hengqin Zhuhai, Guangdong 519031 (CN)

(72) Inventors:
• PENG, Yucai
Macau TCM SCI&TECH Industry Park, Bldg AC2, 1st
FI, Hengqin Zhuhai, Guangdong 519031 (CN)

• LIU, Qi
Macau TCM SCI&TECH Industry Park, Bldg AC2, 1st
FI, Hengqin Zhuhai, Guangdong 519031 (CN)
• LIU, Jun
Macau TCM SCI&TECH Industry Park, Bldg AC2, 1st
FI, Hengqin Zhuhai, Guangdong 519031 (CN)
• LI, Zhigang
Macau TCM SCI&TECH Industry Park, Bldg AC2, 1st
FI, Hengqin Zhuhai, Guangdong 519031 (CN)

(74) Representative: Rentsch Partner AG
Kirchenweg 8
Postfach
8034 Zürich (CH)

Remarks:
The complete document including Reference Table(s) and the Sequence Listing(s) can be downloaded from the EPO website

(54) **THERAPEUTIC NUCLEIC ACID MOLECULE, MIXTURE, AND DRUG, AND USE THEREOF IN TREATING SOLID TUMOR**

(57) The present invention relates to the technical field of gene drugs, and in particular to a therapeutic nucleic acid molecule, mixture, and drug, and use thereof in treating a solid tumor. Provided is a nucleic acid molecule or nucleic acid molecule mixture with a nucleic acid molecule fragment encoding interleukin IL-12 p35 and p40 subunits and a nucleic acid molecule fragment encoding an IL-7 protein as main components. Also provided are nucleic acid molecules comprising the described nucleic acid molecule fragments in different combination forms. The combination forms comprise the fusion of different nucleic acid molecule fragments and the formation of a composition by different nucleic acid molecule fragments in a free form. Upon verification, the provided nucleic acid molecule fragments, whether in the form of free nucleic acid molecule fragments or prepared into a fusion nucleic acid molecule, have shown effective therapeutic effects on solid tumors. Further, an aptamer is used to be compounded with the described nucleic acid molecule composition or fusion nucleic acid molecule to obtain the nucleic acid molecule drug, and a corresponding preparation method is provided, so as to alleviate the present urgent need for drugs in the treatment of solid tumors.

FIG.2

**Description**

**CROSS-REFERENCE TO RELATED APPLICATIONS**

This application claims the priority of the Chinese patent application with the filing date of May 6, 2022 (Application No.: 2022104869114, Invention Title: Therapeutic Nucleic Acid Molecule, Mixture, and Drug, and Use Thereof in Treating Solid Tumor), part of which is incorporated by reference into this application; and the priority of the Chinese patent application with the filing date of May 4, 2023 (Application No.: 2023104938689, Invention Title: Therapeutic Nucleic Acid Molecule, Mixture, and Drug, and Use Thereof in Treating Solid Tumor), the entire contents of which are incorporated by reference into this application in its entirety.

**TECHNICAL FIELD**

The present disclosure relates to the technical field of genetic medicine, and in particular to a therapeutic nucleic acid molecule, mixture, medicament and their uses in the treatment of solid tumors.

**BACKGROUND**

Tumors are clinically divided into solid tumors and non-solid tumors. Solid tumors (or shaped tumors) can be detected through clinical examinations such as X-ray, CT scan, and B-ultrasound, or can be tangible masses palpable by palpation. Tumors that cannot be seen or palpated by X-ray, CT scan, B-ultrasound and palpation, such as leukemia among blood diseases, are non-solid tumors.

There are four mainstream treatments for solid tumors: surgery, chemotherapy, radiotherapy and targeted therapy. These methods can remove, damage, kill, and destroy tumor cells to alleviate the condition. Depending on the type of tumor, one type of treatment may be used, or a combination of treatments may be used. Moreover, tumor immunotherapy has developed rapidly and has gradually become another effective treatment method in addition to the four mainstream tumor treatment methods. Due to the superior properties of nucleic acid molecules, tumor immunotherapy based on the nucleic acid molecule has also been proposed. In brief, treatment based on the nucleic acid molecule takes advantage of the entry of a chemically modified nucleic acid molecule into the cytoplasm, and uses its own nucleotides in the cytoplasm for transcription and expression to produce the protein required by the body. However, currently, therapeutic products based on the nucleic acid molecule are still lacking.

**SUMMARY OF THE DISCLOSURE**

An objective of the present disclosure includes the provision of a nucleic acid molecule or a nucleic acid molecule mixture that has a therapeutic effect on solid tumors, so as to alleviate at least one technical problem existing in the prior art.

In order to achieve at least one of the above objectives of the present disclosure, the following technical solutions are adopted.

In a first aspect, the present disclosure provides a therapeutic nucleic acid molecule or a nucleic acid molecule mixture, the nucleic acid molecule comprises: a nucleic acid molecule fragment (A) encoding an IL-7 protein; and a nucleic acid molecule fragment (B) encoding p35 and p40 subunits of interleukin IL-12.

Nucleic acid or nucleic acid molecule as used herein refers to a polymeric form of nucleotides of any length, including ribonucleotide and/or deoxyribonucleotide. Examples of nucleic acid or nucleic acid molecule include, but are not limited to, single-, double- or multi-stranded DNA or RNA, genomic DNA, cDNA, DNA-RNA hybrid, or a polymer containing purine and pyrimidine bases or other natural, chemically or biochemically modified, non-natural or derived nucleotide bases. The protein or polypeptide of interest encoded by the nucleic acid or nucleic acid molecule may optionally be an encoding sense strand or antisense strand. Nucleic acid or nucleic acid molecule may be naturally occurring, synthetic, recombinant, or any combination thereof.

IL-12 is mainly produced by activation of antigen-presenting cells such as dendritic cells and macrophages. IL-12 binds to the IL-12 receptors IL-12RB1 and IL-12RB2 on the cell membrane, activating the downstream Jak2 and Tyk2, thereby inducing STAT4 phosphorylation and dimerization, which binds to the promoter of the target gene and regulates the gene expression, such as IFN$\gamma$. IL-12 can activate NK cells to express CD69 and CD25, and promote the expansion of NK cells. IL-12 can promote the differentiation and activation of Th1 cells, thereby activating killer CD8$^+$ T cells. IL-12 can promote the differentiation of macrophages into the pro-inflammatory M1 type instead of the anti-inflammatory M2 type, thereby inducing the expression of chemokines CXCL9, CXCL10 and CXCL11 and promoting the recruitment and enrichment of immune cells.

IL-7 is widely expressed in tissues including lymph node, bone marrow, spleen, skin, lung, liver and other tissues. IL-7 activates Jak-Stat5 and PI3K-AKT signaling pathways via interaction with IL-7 receptor, thereby regulating the expression of downstream genes. IL-7 can improve the tumor killing function of NK, NKT, LAK and CD8 [+] T cells. IL-7 can enhance the killing ability of T cells and NK cells against tumor by inducing them to express perforin, IFNγ, FasL, etc. In addition, IL-7 can inhibit the growth of tumor cells by stimulating monocytes to release IL-1β, IL-1α and TNF-α. IL-7 can downregulate the expression of PD-1 on CD8 [+] T cells, thereby reversing T cell exhaustion. IL-7 plays an important role in maintaining the survival and expansion of memory T cells. Additionally, IL-7 can maintain the homeostasis of memory CD4 [+] and CD8 [+] T cell pools after antigen withdrawal.

There are mutual synergistic effects in terms of the biological functions of cytokines. IL-7 can increase the expression of IL-12 receptors on NK cells and T cells, making the latter more sensitive to IL-12. IL-7 can cooperate with IL-12 to increase the expression of IFN-γ. IL-12 and IL-7 can synergistically activate T cells and NK cells and improve their killing ability against tumor cells. IL-12 and IL-7 synergistically induce NK cells to express NKG2E, NKp44, and NKp46, promoting the maturation of NK cells. IL-12 and IL-7 work synergistically to induce the expression of HLA-DR in antigen-presenting cells and promote their antigen-presenting capability. IL-12 and IL-7 work synergistically to enhance T cell expansion and maintain tumor-infiltrating lymphocytes. IL-7 can increase the TCR diversity of tumor-infiltrating CD8 [+] T cells, while IL-12 and IL-7 work synergistically to increase the proportion of a small fraction of T cell clones, thereby enhancing T cell clonality. This synergistic effect activates tumor-infiltrating T cells and enhances anti-tumor activity.

In an alternative embodiment, the nucleic acid molecule further comprises a nucleic acid molecule fragment (C), and the nucleic acid molecule fragment (C) encodes at least one of the following proteins: IFN-α, IFN-β, IFN-γ, GM-CSF, IL15 and IL-2, wherein the nucleic acid molecule fragment (C) encodes IFN-α. IFN-α can improve the antigen presentation ability of dendritic cells and promote their migration to lymph nodes. IFN-α improves the killing capability of immune cells by inducing the release of perforin and granzyme. IFN-α can inhibit the immunosuppressive function and cell expansion ability of Treg and MDSC. IFN-α can promote the differentiation of M1 macrophages. In addition, IFN-α can also exert direct anti-tumor effects by mechanisms such as inhibiting the cell cycle of the tumor cell, promoting terminal differentiation of the cell, and inducing apoptosis.

In an alternative embodiment, based on the biological functions of the cytokines IL-12, IL-7 and IFN-α and the synergy between them, in the field of anti-tumor treatment, in an alternative embodiment, the combined use of these three cytokines is expected to achieve the following main effects: inducing the differentiation and activation of Th1 cells, inducing the maturation of NK cells, activating and promoting the expansion of T cells and NK cells, the capability of killing tumors, and maintaining survival and expansion of memory T cells; promoting the differentiation of macrophages into the pro-inflammatory M1 type, enhancing the antigen-presenting ability of dendritic cells, and promoting the recruitment of immune cells and infiltration into tumor tissues by inducing the expression of chemokines; reversing T cell exhaustion, inhibiting the functions of Treg and MDSC; directly inhibiting the growth of tumor cells and inducing apoptosis.

In an alternative embodiment, the amino acid sequence of IL-7 polypeptide encoded by the nucleic acid molecule fragment (A) is set forth in SEQ ID NO. 42, or comprises an amino acid sequence having at least 80% identity to the amino acid sequence set forth in SEQ ID NO. 42, for example, may be, but is not limited to, an amino acid sequence having at least 80%, 85%, 90%, 95% or 98% identity to the amino acid sequence set forth in SEQ ID NO. 42.

In an alternative embodiment, the nucleotide sequence of the nucleic acid molecule fragment (A) is selected from any one of the nucleotide sequences set forth in SEQ ID NOs. 22 - 24 and SEQ ID NOs. 47 - 49, wherein the nucleotide sequences set forth in SEQ ID NOs. 22 - 24 are RNA sequences, and the nucleotide sequences set forth in SEQ ID NOs. 47 - 49 are DNA sequences.

In an alternative embodiment, the nucleotide sequence of the nucleic acid molecule fragment (A) is selected from any one of the nucleotide sequences having at least 70%, 75%, 80%, at least 90%, at least 95%, or at least 98% identity to the nucleotide sequence set forth in SEQ ID NOs. 22 - 24 and SEQ ID NOs. 47 - 49.

In an alternative embodiment, the amino acid sequence of the p35 subunit polypeptide of IL-12 encoded by the nucleic acid molecule fragment (B) is set forth in SEQ ID NO. 39, or comprises an amino acid sequence having at least 80% identity to the amino acid sequence set forth in SEQ ID NO. 39, for example, may be, but is not limited to, an amino acid sequence having at least 80%, 85%, 90%, 95% or 98% identity to the amino acid sequence set forth in SEQ ID NO. 39.

In an alternative embodiment, the amino acid sequence of the p40 subunit polypeptide of IL-12 encoded by the nucleic acid molecule fragment (B) is set forth in SEQ ID NO. 40, or comprises an amino acid sequence having at least 80% identity to the amino acid sequence set forth in SEQ ID NO. 40, for example, may be, but is not limited to, an amino acid sequence having at least 80%, 85%, 90%, 95% or 98% identity to the amino acid sequence set forth in SEQ ID NO. 40.

In an alternative embodiment, the p35 subunit and p40 subunit polypeptide of IL-12 encoded by the nucleic acid molecule fragment (B) are connected through a linker whose amino acid sequence is set forth in SEQ ID NO. 41 (GSSGGGGSPGGGSS).

In an alternative embodiment, the nucleotide sequence of the nucleic acid molecule fragment (B) is selected from any one of the nucleotide sequences set forth in SEQ ID NOs. 19 - 21 and SEQ ID NOs. 44 - 46, wherein the nucleotide sequences set forth in SEQ ID NOs. 19 - 21 are RNA sequences, and the nucleotide sequences set forth in SEQ ID NOs. 44-46 are DNA sequences.

In an alternative embodiment, the nucleotide sequence of the nucleic acid molecule fragment (B) is selected from any one of the nucleotide sequences having at least 70%, 75%, 80%, at least 90%, at least 95%, or at least 98% identity to the nucleotide sequences set forth in SEQ ID NOs. 19 - 21 and SEQ ID NOs. 44-46.

In an alternative embodiment, the amino acid sequence of IFN-$\alpha$ polypeptide encoded by the nucleic acid molecule fragment (C) is set forth in SEQ ID NO. 43, or comprises an amino acid sequence having at least 80% identity to the amino acid sequence set forth in SEQ ID NO. 43, for example, may be, but is not limited to, an amino acid sequence having at least 80%, 85%, 90%, 95% or 98% identity to the amino acid sequence set forth in SEQ ID NO. 43.

In an alternative embodiment, the nucleotide sequence of the nucleic acid molecule fragment (C) is selected from any one of the nucleotide sequences set forth in SEQ ID NOs. 26 - 28 and SEQ ID NOs. 50 - 52, wherein the nucleotide sequences set forth in SEQ ID NOs. 26 - 28 are RNA sequences, and the nucleotide sequences set forth in SEQ ID NO. 50 - 52s are DNA sequences.

In an alternative embodiment, the nucleotide sequence of the nucleic acid molecule fragment (C) is selected from any one of the nucleotide sequences having at least 70%, 75%, 80%, at least 90%, at least 95%, or at least 98% identity to the nucleotide sequences set forth in SEQ ID NOs. 26 - 28 and SEQ ID NOs. 50 - 52.

In an alternative embodiment, the nucleic acid molecule fragment (A), the nucleic acid molecule fragment (B) and/or the nucleic acid molecule fragment (C) can optimize the mRNA sequence through sequence optimization means to improve the characteristics related to the expression efficacy upon *in vivo* administration: for example, improving mRNA stability, increasing translation efficiency in target tissues, reducing the number of expressed truncated proteins, improving the folding of expressed proteins or preventing their misfolding, reducing the toxicity of expression products, and reducing cell death caused by expressed products, increasing and/or reducing protein aggregation, and obtaining mRNA with improved properties. Goals of sequence optimization also include: optimizing the formulation and delivery characteristics of nucleic acid-based therapeutic agent while maintaining structural and functional integrity; overcoming expression thresholds; increasing expression efficiency, half-life and/or protein concentration; optimizing protein localization; and avoiding adverse biological responses such as immune responses and/or degradation pathways. Means for sequence optimization include: (1) Codon optimization based on codon frequencies in specific organs and/or host organisms to ensure proper folding and proper expression; (2) Adjustment of G/C content to increase mRNA stability or reduce secondary structure; (3) minimizing tandemly repeated codons or base runs that may impair gene construction or expression; (4) customizing transcription and translation control regions; (5) reducing or eliminating problematic secondary structure within the polynucleotide.

"Sequence identity" between two nucleotide sequences indicates the percentage of identical nucleotides between the sequences. "Sequence identity" between two amino acid sequences indicates the percentage of identical amino acids between the sequences.

The term "% identity" or similar terms refers to the percentage of identical nucleotides or amino acids between the sequences to be compared, under optimal alignment. This percentage is purely statistical, and the differences between the two sequences may, but are not necessarily, be randomly distributed over the entire length of the sequences to be compared. Comparison of two sequences is usually performed by comparing the sequences against fragments or "comparison windows" after optimal alignment to identify local regions of corresponding sequences.

In an alternative embodiment, the nucleic acid molecule comprises DNA molecule and/or RNA molecule.

In an alternative embodiment, the DNA molecule comprises stranded DNA molecule and/or circular DNA molecule.

In an alternative embodiment, the RNA molecule comprises mRNA or circular RNA.

In an alternative embodiment, the 5' end and/or 3' end of the nucleic acid molecule fragment has a protective modifying group.

In an alternative embodiment, the nucleic acid molecule fragment is an mRNA fragment, and the 5' end modifying group of the mRNA fragment is selected from ARCA, m7G(5')ppp(5')(2'OMeA) pG, m7G(5')ppp(5')(2'OMeG)pG, m7(3'OMeG)(5')ppp(5')(2'OMeG)pG, m7(3'OMeG)(5')ppp(5')(2'OMeA)pG, mCAP, dmCAP, tmCAP or dmCAP.

In an alternative embodiment, the 3' end protective modifying group of the mRNA fragment is poly(A), and the length of the poly(A) is 50 to 200, optionally 80 to 200.

In an alternative embodiment, the mRNA fragment further comprises a 5' UTR;

In an alternative embodiment, the length of the 5' UTR is optionally 10-200 nucleotides, optionally 15-100 nucleotides;

In an alternative embodiment, the 5'UTR comprises a KOZAK sequence or DNAH2 5'UTR;

In an alternative embodiment, the nucleotide sequence of the KOZAK sequence is set forth in SEQ ID NO. 16;

In an alternative embodiment, the nucleotide sequence of DNAH2 5' UTR is set forth in SEQ ID NO.38;

In an alternative embodiment, the mRNA fragment further comprises 3' UTR;

In an alternative embodiment, the 3' UTR sequence is set forth in SEQ ID.NOs.1-10. SEQ ID.NO.1 originates from creatine kinase (CK), SEQ ID.NO.2 originates from myoglobin, SEQ ID.NO.3 originates from actin (α-actin), SEQ ID.NO.4 originates from albumin, SEQ ID.NO.5 and 7 originate from histamine globulin (α-globin), SEQ ID.NO.6 originates from collagen (Col6a2; collagen, type IV, alpha 2), and SEQ ID.NO.10 originates from hemoglobin HBA2.

In an alternative embodiment, the mRNA sequentially comprises a 5' cap, 5' UTR, ORF, 3' UTR and 3' poly(A) tail from the 5' end to the 3' end.

In an alternative embodiment, based on the provided RNA sequence, one of ordinary skill in the art will be able to obtain the corresponding DNA sequence (e.g., uracil to thymine). Likewise, based on the provided DNA sequence, one of ordinary skill in the art will derive the corresponding RNA sequence (e.g., thymine to uracil). In an alternative embodiment, based on the provided RNA or DNA sequence, one of ordinary skill in the art will be able to obtain the corresponding amino acid sequence.

In an alternative embodiment, one or more uridines in the mRNA are replaced with modified nucleosides. In some embodiments, the modified nucleoside replacing uridine is pseudouridine (ψ), N1-methyl-pseudouridine (m1ψ), or 5-methyl-uridine (m5U).

In a second aspect, the present disclosure provides a first therapeutic nucleic acid molecule composition comprising the nucleic acid molecule mixture according to any one of the preceding embodiments.

In an alternative embodiment, the mass ratio between individual free nucleic acid molecule fragments in the first therapeutic nucleic acid molecule composition is 10:1-1:10; optionally, the mass ratio between individual free nucleic acid molecule fragments can be, for example, but is not limited to 10:1, 7:1, 4:1, 3:1, 2:1, 1:1, 1: 2, 1: 3, 1: 4, 1:7, 1:10.

In a third aspect, the present disclosure provides a therapeutic fusion nucleic acid molecule comprising the nucleic acid molecule according to any one of the preceding embodiments, and the nucleic acid molecule comprises the nucleic acid molecule fragment (A) and the nucleic acid molecule fragment (B). In an alternative embodiment, the nucleic acid molecule further comprises the nucleic acid molecule fragment (C).

In an alternative embodiment, at least any two of the nucleic acid molecule fragments are connected through a linker.

In an alternative embodiment, the mass ratio between individual nucleic acid molecule fragments in the fusion nucleic acid molecule is 10:1-1:10; optionally, the mass ratio between individual free nucleic acid molecule fragments can be, for example, but is not limited to 10:1, 7:1, 4:1, 3:1, 2:1, 1:1, 1: 2, 1: 3, 1: 4, 1: 7, 1:10.

In a fourth aspect, the present disclosure provides a second therapeutic nucleic acid molecule composition comprising a combination of the first therapeutic nucleic acid molecule composition described in the previous embodiments and the therapeutic fusion nucleic acid molecule described in the previous embodiments.

In an alternative embodiment, the mass ratio between individual nucleic acid molecule fragments in the second therapeutic nucleic acid molecule composition is 10:1-1:10; optionally, the mass ratio between individual free nucleic acid molecule fragments can be, for example, but is not limited to 10:1, 7:1, 4:1, 3:1, 2:1, 1:1, 1:2, 1:3, 1:4, 1:7, 1:10.

In a fifth aspect, the present disclosure provides use of the nucleic acid molecule or nucleic acid molecule mixture according to any one of the preceding embodiments, the first therapeutic nucleic acid molecule composition according to the preceding embodiments, the therapeutic fusion nucleic acid molecule according to the preceding embodiments, or the second therapeutic nucleic acid molecule composition according to the preceding embodiments for the preparation of anti-solid tumor drugs or for the evaluation of the efficacy of solid tumor drugs.

In an alternative embodiment, the mass ratio between individual nucleic acid molecule fragments in the nucleic acid molecule or nucleic acid molecule mixture is 10:1-1:10; optionally, the mass ratio between individual free nucleic acid molecule fragments can be, for example, but is not limited to 10:1, 7:1, 4:1, 3:1, 2:1, 1:1, 1:2, 1:3, 1:4, 1:7, 1:10.

In an alternative embodiment, the solid tumor includes epithelial tumor, Hodgkin's lymphoma, non-Hodgkin's lymphoma, prostate tumor, ovarian tumor, renal cell tumor, gastrointestinal tumor, liver tumor, colorectal tumor, hemangioma, mesothelioma, pancreatic tumor, breast tumor, sarcoma, lung tumor, colon tumor, brain tumor, melanoma, small cell lung tumor, neuroblastoma, testicular tumor, carcinoid tumor, adenocarcinoma, glioma, sperm cell carcinoma, retinoblastoma or osteosarcoma.

In a sixth aspect, the present disclosure provides a nucleic acid molecule drug for treating solid tumors, comprising nucleic acid molecule component(s) and a carrier that encapsulates the nucleic acid molecule component(s); the nucleic acid molecule component is selected from the therapeutic nucleic acid molecule or nucleic acid molecule mixture as described in any one of the preceding embodiments, the first therapeutic nucleic acid molecule composition as described in the preceding embodiments, and the therapeutic fusion nucleic acid molecule as described in the preceding embodiments, or the second therapeutic nucleic acid molecule composition as described in the preceding embodiments.

In an alternative embodiment, the mass ratio between individual free nucleic acid molecule fragments is 10:1-1:10; optionally, the mass ratio between individual free nucleic acid molecule fragments can be, for example, but is not limited to 10:1, 7:1, 4:1, 3:1, 2:1, 1:1, 1: 2, 1: 3, 1: 4, 1: 7, 1:10.

In an alternative embodiment, the mass ratio between individual nucleic acid molecule fragments in the fusion nucleic acid molecule is 10:1 to 1:10; optionally, the mass ratio between individual nucleic acid molecule fragments in the fusion nucleic acid molecule can be, for example, but is not limited to 10:1, 7:1, 4:1, 3:1, 2:1, 1:1, 1:2, 1:3, 1:4, 1:7, 1:10.

In an alternative embodiment, the carrier comprises a liposomal nanoparticle.

In an alternative embodiment, each of the free nucleic acid molecule fragments or the fusion nucleic acid molecule in the nucleic acid molecule component is independently encapsulated by the liposomal nanoparticle.

In an alternative embodiment, at least two of the nucleic acid molecule fragments or fusion nucleic acid molecule are independently encapsulated by the liposomal nanoparticle. For example, two of the nucleic acid molecule fragments, or three of the nucleic acid molecule fragments, or fusion nucleic acid molecules are independently encapsulated by the liposomal nanoparticle.

In an alternative embodiment, at least two of the nucleic acid molecule fragments are co-encapsulated by the liposomal nanoparticle. For example, two of the nucleic acid molecule fragments, or three of the nucleic acid molecule fragments are co-encapsulated by the liposomal nanoparticle.

In an alternative embodiment, at least one of the nucleic acid molecule fragments and one fusion nucleic acid molecule are co-encapsulated by the liposomal nanoparticle.

In an alternative embodiment, the liposomal nanoparticle comprises 20% to 50% cationic lipid, for example but is not limited to 20%, 25%, 30%, 35%, 40%, 45% or 50%; 20%-50% DOPG, for example but is not limited to 20%, 25%, 30%, 35%, 40%, 45% or 50%; 5%-20% cholesterol, for example but is not limited to 5%, 10%, 15% or 20%; and 1%-5% PEG-DMG, for example but is not limited to 1%, 2%, 3%, 4% or 5%, on a molar percentage basis.

In an alternative embodiment, the liposomal nanoparticle comprises 50% Dlin-MC3-DMA, 10% DOPG, 38.5% cholesterol, and 1.5% PEG-DMG on a molar percentage basis.

In an alternative embodiment, the nucleic acid molecule drug further comprises a therapeutic protein or a therapeutic chemical pharmaceutical component.

In an alternative embodiment, the therapeutic protein comprises atezolizumab.

In a seventh aspect, the present disclosure provides a method for preparing a nucleic acid molecule drug according to any one of the preceding embodiments: dissolving the nucleic acid molecule fragment(s) and/or the fusion nucleic acid molecule in a buffer to obtain an aqueous phase, measuring individual lipid components of the liposomal nanoparticle and dissolving said components in an organic solvent to obtain an organic phase, mixing the aqueous phase and the organic phase, and then removing the organic phase to obtain the nucleic acid molecule drug.

In an alternative embodiment, the volume ratio of the aqueous phase and the organic phase is 1:2-4, optionally 1:3.

In an alternative embodiment, the buffer comprises citrate buffer or sodium acetate, optionally citrate buffer.

In an alternative embodiment, the pH of the buffer is 3 to 7, optionally 4.

In an alternative embodiment, the concentration of the free nucleic acid molecule fragment or fusion nucleic acid molecule in the aqueous phase is 0.05 mg/mL-0.5 mg/mL, optionally 0.1 mg/mL.

In an alternative embodiment, the organic solvent is selected from C1 to C4 lower alcohols, optionally anhydrous ethanol.

In an alternative embodiment, the concentration of the lipid component in the organic phase is 5 mg/mL-7 mg/mL, optionally 6 mg/mL.

In an alternative embodiment, the aqueous phase and the organic phase are mixed by microfluidics, and the organic solvent is filtered using tangential flow.

In an alternative embodiment, the flow rate of the microfluidics is >3 ml/min, further optionally 12 mL/min.

In an alternative embodiment, the method further comprises a concentration step after mixing, wherein the concentration step allows the final concentration of the free nucleic acid molecule fragment(s) and/or the fusion nucleic acid molecule to be from 50 $\mu$g/mL to 200 $\mu$g/mL, optionally 100 $\mu$g/mL.

In an eighth aspect, the present disclosure provides a pharmaceutical composition, which comprises the nucleic acid molecule drug described in any one of the preceding embodiments, or the nucleic acid molecule drug prepared by using the preparation method described in any one of the preceding embodiments.

In an alternative embodiment, the pharmaceutical composition further comprises a protein drug selected from the group consisting of at least one of anti-PD-1 antibody, anti-PD-L1 antibody, anti-CTLA-4 antibody, anti-DC20 antibody, anti-Her2 antibody, anti-CD33 antibody, anti-CD52 antibody, anti-VEGFR antibody, anti-EGFR antibody, anti-RANKL antibody, anti-CD30 antibody, anti-VEGFR2 antibody, anti-GD2 antibody, anti-CD38 antibody, anti-CD22 antibody and anti-CD33 antibody.

In an alternative embodiment, the pharmaceutical composition further comprises at least one of atezolizumab, nivolumab, pembrolizumab, pidilizumab, durvalumab, avelumab and ipilimumab.

Compared with the prior art, the beneficial effects of the present disclosure include:

The nucleic acid molecule or nucleic acid molecule mixture provided by the present disclosure, wherein the main components are the nucleic acid molecule fragments encoding IL-7 protein and encoding p35 and p40 subunits of interleukin IL-12, can fully activate a variety of immune cell activities to achieve effective treatment of solid tumors.

The present disclosure also provides a nucleic acid molecule containing the above nucleic acid molecule fragments in different combination forms. The combination forms include the fusion of the nucleic acid molecule fragments, and a composition formed by the free nucleic acid molecule fragments. It has been verified that both types of the nucleic acid molecule fragments provided by the present disclosure, whether in the form of free nucleic acid molecule fragments or prepared into a fusion nucleic acid molecule, have shown effective therapeutic effects on solid tumors.

The present disclosure further uses an aptamer for complexing with the above-mentioned nucleic acid molecule composition or fusion nucleic acid molecule to obtain a nucleic acid molecule drug, and provides a corresponding preparation method, in order to alleviate the current urgent need for drugs in the treatment of solid tumors.

## DESCRIPTION OF THE DRAWINGS

In order to more clearly explain the specific embodiments of the present disclosure or the technical solutions in the prior art, the drawings that need to be used in the description of the specific embodiments or the prior art will be briefly introduced below. Apparently, the drawings in the following description illustrate some embodiments of the present disclosure. For those of ordinary skill in the art, other drawings can be obtained based on these drawings without exerting inventive efforts.

Figure 1 is a schematic diagram of the tumor inoculation positions in each group of mouse experiments in the Examples of the present disclosure;
Figure 2 is a graph showing changes in tumor volume in five groups of experiments in Example 1 of the present disclosure;
Figure 3 is a graph showing changes in tumor volume in five groups of experiments in Example 3 of the present disclosure;
Figure 4 is a graph showing changes in tumor volume in four groups of experiments in Example 4 of the present disclosure;
Figure 5 is a physical diagram of the mice in Example 5 of the present disclosure;
Figure 6 is a graph showing changes in tumor volume in three groups of experiments in Example 5 of the present disclosure;
Figure 7 illustrates the detection result of the proportion of leukocytes infiltrating in the tumor in Example 6 of the present disclosure;
Figure 8 is a graph showing the tumor volume as a function of injection days in each experimental group in Example 18 of the present disclosure;
Figure 9 illustrates the changes in tumor volume of each mouse in each experimental group in Example 18 of the present disclosure;
Figure 10 is a graph showing the changes in tumor volume of each experimental group in Example 19 of the present disclosure;
Figure 11 is a graph showing the relative tumor inhibition rate of each experimental group in Example 20 of the present disclosure;
Figure 12 is a graph showing the tumor volume as a function of injection days in each experimental group in Example 20 of the present disclosure;
Figure 13A and Figure 13B illustrate the changes in tumor volume of each mouse in each experimental group in Example 20 of the present disclosure;
Figures 14 is a graph showing the tumor volume as a function of injection days in each experimental group in Example 21 of the present disclosure;
Figures 15 illustrates the changes in tumor volume of G1 to G6 mice in Example 22 of the present disclosure.

## DETAILED DESCRIPTION

The technical solutions of the present disclosure will be clearly and fully described below with reference to the Examples. Apparently, the described Examples are part of the Examples of the present disclosure, rather than all the Examples. Based on the Examples of the present disclosure, all other Examples obtained by those of ordinary skill in the art without inventive efforts fall within the scope of protection of the present disclosure.

In order to demonstrate that the therapeutic nucleic acid molecule or nucleic acid molecule mixture provided by the present disclosure have a therapeutic effect on solid tumors, the present disclosure constructs a method for evaluating the therapeutic effect on solid tumors. The specific steps were as follows:

1. Construction of mouse CT26 tumor model

Selection of experimental animals (1) strain: BALB/c, (2) level: SPF level, (3) age: 7.6-8.7 weeks, (4) gender:

female.

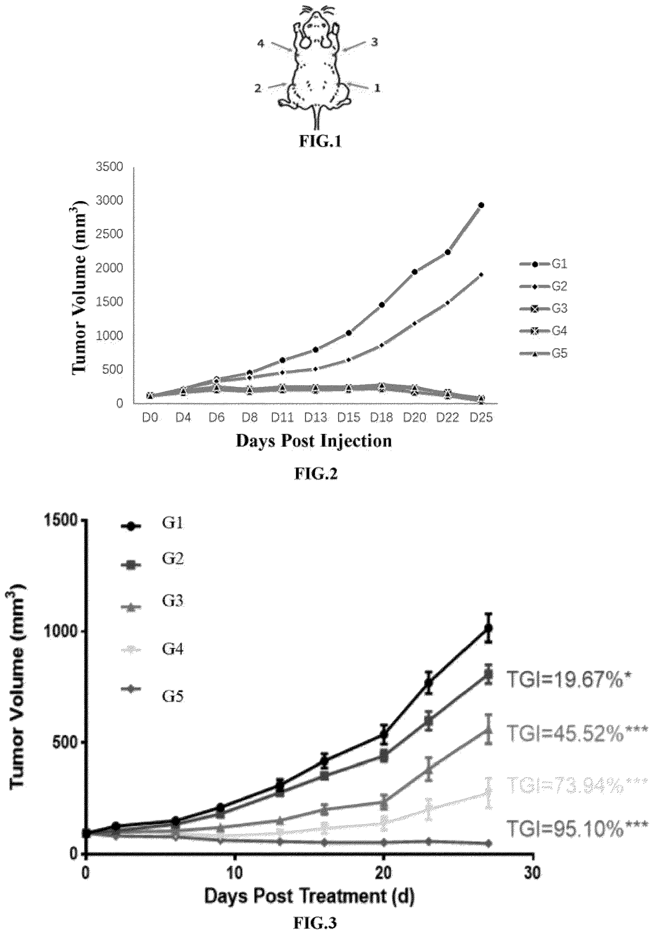

Tumor inoculation: CT26.WT cells were resuscitated and subcultured, and the recovery passage was N+8. CT26.WT cells in the logarithmic growth phase (inoculation passage N+11) were collected; the culture medium was removed and the cells were washed twice with PBS before inoculation. Inoculation volume: $1 \times 10^6$ cells/100 $\mu$L /mouse. The inoculation position was Position 1 as shown in Figure 1.

2. Construction of mouse KM12 tumor model

Selection of experimental animals (1) strain: SCID mouse, (2) level: SPF level, (3) age: 10 weeks, (4) gender: female.

Tumor inoculation: KM-12 cells were resuscitated and subcultured, and the recovery passage was N+8. KM-12 cells in the logarithmic growth phase (inoculation passage N+11) were collected; the culture medium was removed and the cells were washed twice with PBS before inoculation. Inoculation volume: $5 \times 10^6$ cells/200 $\mu$L/mouse. The inoculation position was Position 1 as shown in Figure 1.

3. Construction of mouse Cal27 tumor model

Selection of experimental animals (1) strain: BALB/c nude mouse, (2) level: SPF level, (3) age: 4-6 weeks, (4) gender: male.

Tumor inoculation: Cal27 cells were resuscitated and subcultured, and the recovery passage was N+8. Cal27 cells in the logarithmic growth phase (inoculation passage N+11) were collected; the culture medium was removed and the cells were washed twice with PBS before inoculation. Inoculation volume: $5 \times 10^9$ cells/L/mouse. The inoculation position was Position 2 as shown in Figure 1.

4. Construction of mouse NCI-N87 tumor model

Selection of experimental animals (1) strain: SCID, (2) level: SPF level, (3) age: 11 weeks, (4) gender: female.

Tumor inoculation: NCI-N87 cells were resuscitated and subcultured, and the recovery passage was N+8. NCI-N87 cells in the logarithmic growth phase (inoculation passage N+11) were collected; the culture medium was removed and the cells were washed twice with PBS before inoculation. Inoculation volume: $3.0 \times 10^6$ cells/ 200 $\mu$L/mouse. The inoculation position was Position 1 as shown in Figure 1.

5. Construction of mouse A375 tumor model

Selection of experimental animals (1) strain: SCID, (2) level: SPF level, (3) age: 6-8 weeks, (4) gender: female.

Tumor inoculation: A375 cells were resuscitated and subcultured, and the recovery passage was N+8. A375 cells in the logarithmic growth phase (inoculation passage N+11) were collected; the culture medium was removed and the cells were washed twice with PBS before inoculation. Inoculation volume: $5 \times 10^6$ cells/ 200 $\mu$L /mouse. The inoculation position was Position 1 as shown in Figure 1.

6. Construction of mouse NCI-H1975 tumor model

Selection of experimental animals (1) strain: NSG, (2) level: SPF level, (3) age: 10 weeks, (4) gender: female.

Tumor inoculation: NCI-H1975 cells were resuscitated and subcultured, and the recovery passage was N+8. NCI-H1975 cells in the logarithmic growth phase (inoculation passage N+11) were collected; the culture medium was removed and the cells were washed twice with PBS before inoculation. Inoculation volume: $1 \times 10^6$ cells/100 $\mu$L /mouse. The inoculation position was Position 1 as shown in Figure 1.

7. Construction of mouse MDA-MB-231 tumor model

Selection of experimental animals (1) Species: mouse; (2) Strain: huHSC-NCG-hIL15 (T038070); (3) Level: SPF level; (4) Age: 16.7 weeks (age at the time of inoculation); (5) Gender: female.

Identification of humanized mouse: 8.7 weeks after huHSC-NCG-hIL15 immune reconstitution, the following indicators of the mouse peripheral blood were analyzed by flow cytometry: hCD45+, hCD3+, CD4, CD8, NK (hCD56+, hCD16+). The average proportion of hCD45+ cells in viable cells was 24.82%, and the average proportion of hCD56+/hCD45+ was 10.8%. It was determined that the humanized model was successfully constructed; and cell expansion was performed.

Tumor inoculation: MDA-MB-231 cells were resuscitated and subcultured, and the recovery passage was N+23.

MDA-MB-231 cells in the logarithmic growth phase were collected 12.5 weeks after immune reconstitution (inoculation passage was N+33); the culture medium was removed and the cells were washed twice with PBS before inoculation. Inoculation volume: $5 \times 10^6$ cells/100 $\mu$L/mouse (Matrigel 1:1). The inoculation position was Position 3 as shown in Figure 1.

8. Animal welfare

During the experiment, tumor size was observed, mouse body weight was weighed, and clinical pathological assessment was performed. Clinical scores (body weight, mouse posture, mobility, hair, skin, etc.) were evaluated based on clinical observations. If the experimental mouse shows the following indicators, the animals will be euthanized: (1) When the tumor volume of a single mouse exceeds 3000 mm$^3$, a single mouse will euthanized; (2) Persistent loose stools; (3) Sluggish movement (cannot eat or drink); (4) Arched back, lying on one side; (5) Reduced activity, symptoms of muscle atrophy; (6) Difficulty in breathing; (7) Progressive hypothermia; (8) Paralysis, spasm; (9)) Continuous bleeding; (10) The animal is unable to move normally due to large tumors or other reasons; (11) The animal is unable to move normally due to severe ascites or increased abdominal girth.

9. Index calculation formula

The calculation method of tumor volume is: tumor volume (mm$^3$) = 0.5$\times$long diameter of the tumor $\times$short diameter of the tumor$^2$.

TGI $_{TV}$ (relative tumor inhibition rate) calculation formula:

$$RTV_n = \frac{V_{nt}}{V_{n0}};$$

$$TGI_{TV} = \left(1 - \frac{mean\ RTV_{treat}}{mean\ RTV_{vehicle}}\right) \times 100\% ;$$

$V_{nt}$: Tumor volume of mouse numbered n on day t;

$V_{n0}$: Tumor volume of mouse numbered n on day 0;

$RTV_n$: Relative tumor volume of mouse numbered n on day t;

mean $RTV_{treat}$: Average RTV of the treatment group;

mean $RTV_{vehicle}$: Average RTV of the vehicle group;

TGI $_{TW}$ (tumor weight change) calculation formula:

$$TGI_{TW} = \left(1 - \frac{mean\ TW_{treat}}{mean\ TW_{vehicle}}\right) \times 100\% ;$$

mean $TW_{treat}$: The average tumor weight of mouse in the treatment group at the endpoint;

mean $TW_{vehicle}$: The average tumor weight of mouse in the vehicle group at the endpoint.

10. Statistical analysis

Experimental results were expressed as mean $\pm$ SEM. Comparison between two groups of samples was performed using independent sample T test (T - Test). The data was analyzed using SPSS, and $P < 0.05$ was considered a significant difference. The drawing software was Graphpad prism.

The present disclosure provides a method for preparing a lipid nanoparticle containing mRNA. Unless otherwise specified, the mRNA used for injection administration in the following examples all adopts the administration method of lipid nanoparticle obtained by independently encapsulating cationic lipid. The preparation method of lipid nanoparticle independently encapsulated with cationic lipid refers to Example 8 or Example 15. The preparation method of lipid nanoparticle co-encapsulated with cationic lipid refers to Example 7, 9, 16 or 17. Those skilled in the art can refer to the above preparation method for preparing a lipid nanoparticle according to the type and quantity of the nucleic acid molecule. In the following examples, the nucleotide sequences of SEQ ID NOs. 11-15 and SEQ ID NO. 25 are of mouse origin, while the nucleosides of SEQ ID NOs. 17- 24 and SEQ ID NOs. 26-29 are of human origin.

It should be noted that the structure of the mRNA containing an open reading frame used in the following examples sequentially comprises a 5' cap, 5' UTR, ORF, 3' UTR and 3' poly (A) tail from the 5' end to the 3' end. The 5' cap is; the sequence of the 5' UTR is set forth in SEQ ID NO. 38; the ORF is, for example, selected from any one of the aforementioned SEQ ID NOs. 11-15 and SEQ ID NOs. 17-29; 3' UTR sequence is set forth in SEQ ID NO. 10; and the 3' poly(A) tail length is: 100A. All uridines in the mRNA sequence are replaced by N1-methyl-pseudouridine (m1ψ). *In situ* injection administration herein refers to intratumoral injection administration (intratumoral injection administration means injection administration inside the tumor, that is, injecting a therapeutic agent at any location that contacts the tumor).

**Example 1**

This Example examined the impact of a combination of mRNAs encoding different polypeptides on tumor inhibition rate.

Five groups of experiments were conducted using the above CT26 tumor model as the experimental model, with the number of mice in each group being 8.

Group 1.1 (G1) was administered with normal saline at a frequency of twice a week for a period of 3 weeks. Group 1.2 (G2) was administered with a mixture consisting of mRNA (the nucleotide sequence is set forth in SEQ ID NO. 14) encoding IL-12 p35 and p40 subunits, mRNA encoding IL-7 polypeptide (the nucleotide sequence is set forth in SEQ ID NO. 15) and mRNA encoding GM-CSF polypeptide (the nucleotide sequence is set forth in SEQ ID NO. 25).

Group 1.3 (G3) was administered with a mixture consisting of mRNA encoding IL-12 p35 and p40 subunits (the nucleotide sequence is set forth in SEQ ID NO. 14), mRNA encoding IL-7 polypeptide (the nucleotide sequence is set forth in SEQ ID NO. 15), mRNA encoding IFN-α polypeptide (the nucleotide sequence is set forth in SEQ ID NO. 13) and mRNA encoding GM-CSF polypeptide (the nucleotide sequence is set forth in SEQ ID NO. 25). Group 1.4 (G4) was administered with a mixture consisting of mRNA encoding IL-12 p35 and p40 subunits (the nucleotide sequence is set forth in SEQ ID NO. 14), mRNA encoding IL15 polypeptide (the nucleotide sequence is set forth in SEQ ID NO. 11), mRNA encoding IFN-α polypeptide (the nucleotide sequence is set forth in SEQ ID NO. 13) and mRNA encoding GM-CSF polypeptide (the nucleotide sequence is set forth in SEQ ID NO. 25). Group 1.5 (G5) was administered with a mixture consisting of mRNA encoding IL-12 p35 and p40 subunits (the nucleotide sequence is set forth in SEQ ID NO. 14), mRNA encoding IL-7 polypeptide (the nucleotide sequence is set forth in SEQ ID NO. 15) and mRNA encoding IFN-α polypeptide (the nucleotide sequence is set forth in SEQ ID NO. 13).

The administration frequency of the mRNA mixture was once a week, and the administration period was 3 weeks. The administration method for the 5 groups of mice was *in situ* injection, wherein the total dose of mRNA in each group was 0.12mg/kg, and different RNAs within each group were administered in equal amounts. 25 days after grouping was set as the endpoint of the experiment, and the impact on tumor was tested for the five groups of experiments. Verified by the above calculation method, the relative tumor inhibition rate ($TGI_{TV}$) of the mixture of IL-12+IL-7+GM-CSF (G2 group) on mouse colon cancer cell CT26 was 36.46%; the relative tumor inhibition rates ($TGI_{TV}$) of the mixture of IL-12+IL-7+ IFN-α+GM-CSF (G3 group), the mixture of IL-12+IL15+IFN-α+GM-CSF (G4 group), and the mixture of IL-12+IL-7+IFN-α (G5 group) on mouse colon cancer cell CT26 were 98.56%, 97.67%, and 97.07%, but there was no significant difference between the relative tumor inhibition rates ($TGI_{TV}$) of the G3 to G5 groups (P>0.05). The results of the changes in tumor volume with the injection days are shown in Figure 2.

In addition, the present disclosure also conducted other component screening for the three-component mRNA mixture IL-12+IL-7+IFN-α. When IFN-α in the three-component mRNA mixture IL-12+IL-7+IFN-α was replaced with IFN-β or IFN-γ, the resulting relative tumor inhibition rates ($TGI_{TV}$) were 67% and 75% respectively, which were slightly lower than IFN-α.

**Example 2**

This Example examined the ability of different mRNA sequences of the same polypeptide fragment to express the polypeptide fragment *in vitro.*

In this Example, the above three mRNAs encoding IL-7 polypeptide (sequences are set forth in SEQ ID NO. 22, SEQ ID NO. 23 and SEQ ID NO. 24 respectively), three mRNAs encoding IL-12 polypeptide (sequences are set forth in SEQ ID NO. 19, SEQ ID NO. 20 and SEQ ID NO. 21 respectively) and three mRNAs encoding IFN-α polypeptide (the sequences are set forth in SEQ ID NO. 26, SEQ ID NO. 27 and SEQ ID NO. 28 respectively) prepared by *in vitro* transcription method were transfected into HEK293 cells respectively. Firstly, HEK293 cells were plated at a density of $4\times10^5$ cells/ml. After 24 hours, the cells were transfected when the cell confluence reached approximately 80%. During transfection, the transfection system comprising 2 μg of mRNA and transfection reagent Lipofectamine MessagerMAX (ThermoFisher Scientific) was added to the HEK293 cells in 1 well of the 6-well plate. The specific transfection procedure was performed according to the transfection reagent product instructions.

After 24 hours, the cell supernatant and cell lysate were collected respectively, and the level of protein expressed by mRNA was detected by ELISA.

| mRNA sequence encoding IL-12 polypeptide | the amount of relative expression |
|---|---|
| SEQ ID NO. 19 | 1.5 |
| SEQ ID NO. 20 | 1 |
| SEQ ID NO. 21 | 0.9 |

| mRNA sequence encoding IL-7 polypeptide | the amount of relative expression |
|---|---|
| SEQ ID NO. 22 | 1.5 |
| SEQ ID NO. 23 | 1 |
| SEQ ID NO. 24 | 0.8 |
| mRNA sequence encoding IFN-α polypeptide | the amount of relative expression |
| SEQ ID NO. 26 | 1.6 |
| SEQ ID NO. 27 | 0.8 |
| SEQ ID NO. 28 | 1 |

For the three mRNA sequences encoding IL-12 polypeptide, the three mRNA sequences encoding IL-7 polypeptide, and the three mRNA sequences encoding IFN-α polypeptide listed in this disclosure, there was no significant difference in terms of such indicators as commonly used codon frequency, GC content, mRNA secondary structure of the gene (such as mRNA free energy), unstable sequences of cis-acting mRNA, RNase cutting sites and repeating units, etc. However, it can be seen from the transcription results that the expression level of IL-12 in cells transfected with the IL-12 mRNA sequence set forth in SEQ ID NO. 19 was higher than that of the IL-12 mRNA sequences set forth in SEQ ID NO. 20 and SEQ ID NO. 21; the expression level of IL-7 in cells transfected with the IL-7 mRNA sequence set forth in SEQ ID NO. 22 was higher than that of the IL-7 mRNA sequences set forth in SEQ ID NO. 23 and SEQ ID NO. 24; and the expression level of IFN-α in cells transfected with the IFN-α mRNA sequence set forth in SEQ ID NO. 26 was higher than that of the IFN-α mRNA sequences set forth in SEQ ID NO. 27 and SEQ ID NO. 28.

In the present disclosure, when any one of the three sequences of IL-12 mRNA was employed for the ORF of IL-12 mRNA in the three-component mixture of mRNA (IL-12+IL-7+IFN-α), and the dosage was adjusted according to the expression level of each IL-12 mRNA sequence; the effects of tumor regression, relative tumor inhibition rate ($TGI_{TV}$), immune memory, and combination with anti-tumor drugs were not affected. These effects are also applicable to both IL-7 and IFN-α.

**Example 3**

This Example examined the impact of different dosages on the tumor inhibitory effect.

The above mouse MDA-MB-231 tumor model was employed as the experimental model. On the 16th day after construction (14.8 weeks after immune reconstitution), 30 humanized tumor-bearing mice were selected and randomly divided into 5 groups according to tumor volume, with 6 mice in each group. The day of grouping was defined as D0, and administration started on the same day of grouping (D0). The remainder of the mice upon grouping was euthanized.

Group 3.1 (G1) dosage regimen: normal saline.

Group 3.2 (G2) dosage regimen: 5mg/kg Tecentriq (positive drug).

Group 3.3 (G3) dosage regimen: 0.032mg/kg mRNA three-component mixture (IL-12+IL-7+IFN-α, SEQ ID NO. 19 + SEQ ID NO. 22 + SEQ ID NO. 26).

Group 3.4 (G4) dosage regimen: 0.16mg/kg mRNA three-component mixture (IL-12+IL-7+IFN-$\alpha$, SEQ ID NO. 19 + SEQ ID NO. 22 + SEQ ID NO. 26).

Group 3.5 (G5) dosage regimen: 0.8mg/kg mRNA three components mixture (IL-12+IL-7+IFN-$\alpha$, SEQ ID NO. 19 + SEQ ID NO. 22 + SEQ ID NO. 26). The changes in tumor volume for mice of G1 to G5 are shown in Figure 3.

According to statistical analysis of tumor volume data at the time of drug withdrawal, compared with the control group: Tecentriq (TGI$_{TV}$ =19.67%) in the G2 group can significantly inhibit tumor growth (P<0.05*); low-dose G3 group (TGI$_{TV}$ =45.52%), G4 group (TGI$_{TV}$ =73.94%) and G5 group (TGI$_{TV}$ =95.10%) can significantly inhibit tumor growth (P<0.001***).

The pharmacodynamic effects of different doses of the subject mRNA three-component mixture and the positive drug Tecentriq in the tumor model of huHSC-NCG-hIL15 mouse subcutaneously loaded with the MDA-MB-231 breast cancer were evaluated in this experiment. The above experimental data showed that under the current test system, compared with the control group G1, the positive drug Tecentriq showed significant tumor inhibition effect at the tumor growth stage (TGI $_{TV}$=19.67%, P=0.021*). The subject mRNA three-component mixture at low, medium and high doses all showed significant tumor inhibitory effects in terms of tumor volume compared with the control group G1.

**Example 4**

The anti-tumor effect of (1) a single drug containing three components of IL-12+IL-7+IFN-$\alpha$ (SEQ ID NO. 14+ SEQ ID NO. 15+ SEQ ID NO. 13); and (2) combined administration of this single drug and a protein drug (taking anti-PD -1 antibody as an example) was tested in this Example.

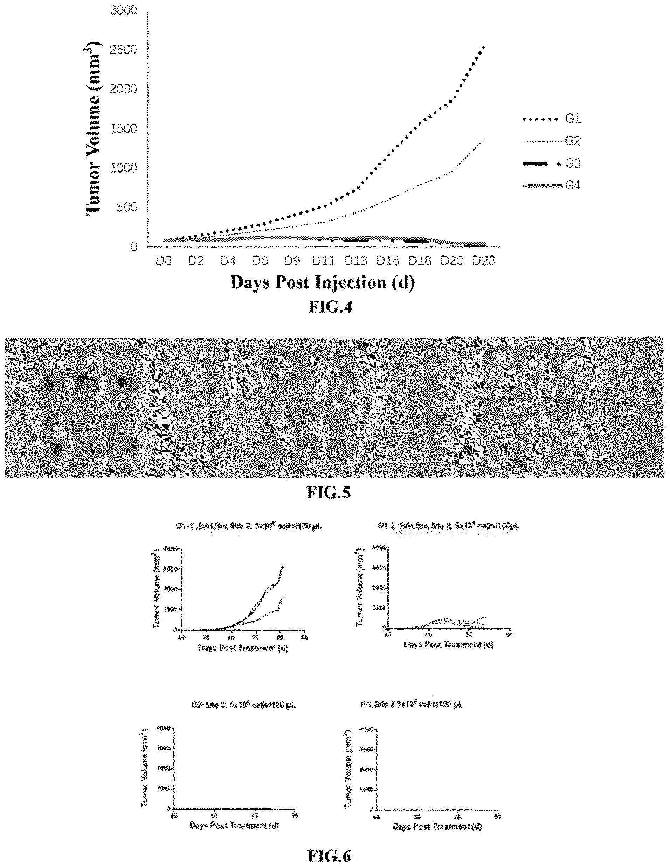

In this experiment, the wild-type BALB/c mouse model was selected, and the mouse colon cancer cell CT26 was inoculated. The following was performed three times at 10 days, 17 days, and 24 days after tumor cell inoculation: (1) intratumoral injection of the above-mentioned single drug; and (2) the combined administration of a single drug and an anti -PD-1 antibody, which were compared with (3) an anti-PD-1 antibody-positive drug. The results were shown in Figure 4, wherein the relative tumor inhibition rate (TGI$_{TV}$) of the single drug containing three different components of mRNA, and its combined use with anti-PD-1 antibody on mouse colon cancer cell CT26 in the wild-type BALB/c mouse model was 99.06% and 98.39% respectively, while the relative tumor inhibition rate of the anti-PD-1 antibody-positive drug was 47.78%. From the above results, it can be concluded that when the single drug formed by the three components of IL-12+IL-7+IFN-$\alpha$ and its combination with anti-PD-1 antibody were examined and the differences in the anti-tumor activity against CT26 mouse colon cancer cells were analyzed, from the perspective of the relative tumor inhibition rate (TGI $_{TV}$) of the results of this experiment, compared with the control group of normal saline (G1), the relative tumor inhibition rate (TGI$_{TV}$) resulting from the single drug formulated with the mixture of IL-12+IL-7+IFN-$\alpha$ (G3) was 99.06%, and its anti-tumor activity was excellent, so when combined with the anti- PD-1antibody (G4, TGI$_{TV}$ was 98.39%), it failed to show the combined synergistic effect with the anti-PD-1 antibody. However, when the drug formulated with the mixture of IL-12+IL-7+IFN-$\alpha$ was used alone, its anti-tumor activity was significantly better than that of the anti-PD-1 antibody alone (G2, TGI$_{TV}$ was 47.78%), and their relative tumor inhibition rates (TGI$_{TV}$) demonstrated a significant difference.

**Example 5**

This Example examined whether the mouse with tumor regression after injection of an LNP preparation containing three components of IL-12+IL-7+IFN-$\alpha$ can produce immune memory.

In this Example, mice with tumor regression were selected, and mice were re-inoculated with colon cancer cells CT26. After tumor cell inoculation, the tumor size was observed on D0, D4, D7, D11, D13, D15, D18, D20, D22, D25, D27, D32, and D34 until 81 days after the first dosing of the animal. The results are shown in Figures 5 and 6.

G1 represented 6 mice that were not injected with the three components of IL-12+IL-7+IFN-$\alpha$; G2 represented 6 mice that were injected with the three components of IL-12+IL-7+IFN-$\alpha$ combined with anti-PD-1 antibody, and had tumor regression; G3 represented 6 mice that were injected with the mixture of IL-12+IL-7+IFN-$\alpha$ alone, and had tumor regression.

G1 was divided into G1-1 and G1-2. G1-1 was only inoculated with mouse colon cancer cell CT26. In addition to inoculation of mouse colon cancer cell CT26, G1-2 was injected with IL-12+IL-7+IFN-$\alpha$ three components on D15.

The results showed: 1) In G1-1 mice that were not injected with the three components of IL-12+IL-7+IFN-$\alpha$, the tumor volume reached 111.84$\pm$1.86 mm$^3$ after 11 days, and the tumor volume reached 2702.94$\pm$485.22 mm$^3$ at the endpoint of the experiment;

2) G1-2 mice were injected with three components of IL-12+IL-7+IFN-$\alpha$ on day D15. The tumor volume reached 99.71$\pm$8.26 mm$^3$ after 11 days, and the tumor volume reached 249.38 + 162.09 mm$^3$ at the endpoint of the experiment;

3) G2 and G3 mice that had tumor regression and were re-inoculated with tumor cells still showed no tumor growth at the endpoint of the test.

From the above results, it can be concluded that after injecting the three components of IL-12+IL-7+IFN-$\alpha$, the effect of tumor regression can be achieved, and after the tumor subsided, re-inoculation of tumor cells can lead to tumor growth inhibition, indicating that long-term immune memory has been established in animals after injection of IL-12+IL-7+IFN-$\alpha$.

**Example 6**

This Example examined the proportion of leukocytes infiltrating in the tumor upon simultaneous injection of three components, murine IL-12 + murine IL-7 + murine IFN-$\alpha$ (nucleotide sequences are set forth in SEQ ID NO. 14, SEQ ID NO. 15 and SEQ ID NO. 13) into the local tumor area.

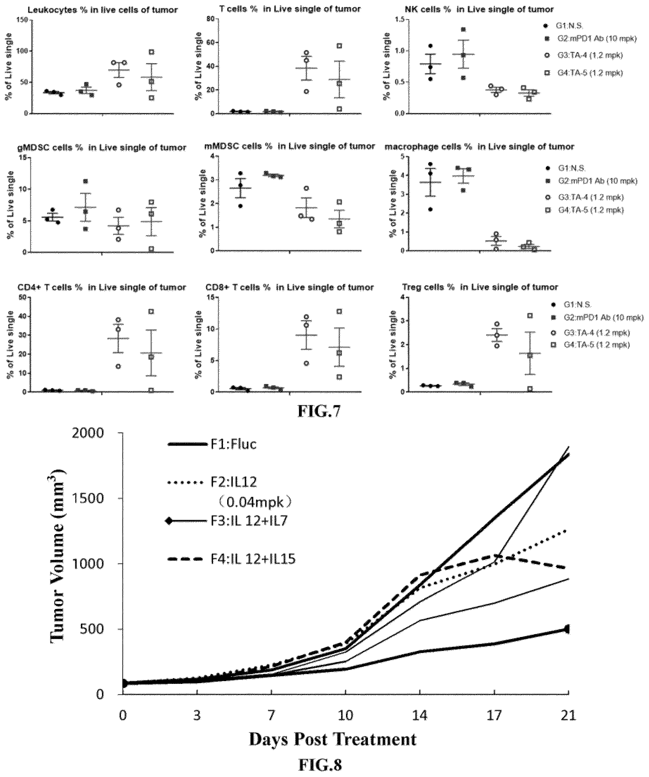

In this Example, a wild-type BALB/c mouse model was selected, and the mouse colon cancer cell CT26 was inoculated. A preparation containing the above three components (G3 and G4 groups) was injected into the tumor twice consecutively 10 days and 17 days after the tumor cell inoculation, which was compared with m PD-1 positive drug (G2 group). The positive drug was injected intraperitoneally, three consecutive times on 10 days, 14 days and 17 days after tumor cell inoculation. The G3 group represented a three-component preparation prepared according to the method described in Example 8, and G4 group represented a three-component preparation prepared according to the method described in Example 7. The results are shown in Figure 7. When the three components of IL-12+IL-7+IFN-$\alpha$ were administered, there were significant differences in terms of T cells, NK cells, gMDSC, mMDSC, macrophages, CD4$^+$T and CD25$^+$CD4$^+$ cells infiltrating in the tumor compared to the normal saline injection group. From the above results, it can be concluded that the proportion of infiltrating leukocytes in the tumor of G3 group and G4 group (the mixture of IL-12+IL-7+IFN-$\alpha$) had an upward trend compared with the G1 (normal saline) and G2 (m PD-1 positive drug) groups. The proportion of T cell infiltration (including CD4$^+$ helper T cells and CD8$^+$ killer T cells) in the tumor showed a larger increase for G3 group and G4 group, consistent with the tumor inhibition results in the pharmacodynamic experiment; and the results from the pharmacodynamic experiment were relatively consistent between G3 group and G4 group.

To evaluate the effect resulting from intratumoral injection of a three-component mixture of mRNA (IL-12+IL-7+IFN-$\alpha$) in various cancer types, five xenograft mouse models were established as previously described: KM12(CRC), Cal27 (head and neck cancer), NCI-N87 (gastric cancer), A375 (melanoma) and NCI-H1975 (NSCLC). Mice bearing tumors of KM12 (CRC), Cal27 (head and neck cancer), NCI-N87 (gastric cancer), A375 (melanoma) and NCI-H1975 (NSCLC) received a three-component mixture of mRNA (IL-12+IL-7+IFN-$\alpha$ (the nucleotide sequences are set forth in SEQ ID NO. 19, SEQ ID NO. 22 and SEQ ID NO. 26)). There were significant differences in terms of T cells, NK cells, gMDSC, mMDSC, macrophages, CD4+T and CD25+CD4+ cells infiltrated in the tumor between the individual tumor model mice tested and the saline injection group.

**Example 7**

This Example provides a method for preparing lipid nanoparticles that simultaneously encapsulate mRNA encoding three polypeptides, namely IL-12 p35 and p40 subunits, IL-7 polypeptide and IFN-$\alpha$ polypeptide. The lipid component of the lipid nanoparticle comprises Dlin-MC3-DMA 50%, DOPG 10%, cholesterol 38.5% and PEG-DMG 1.5% in molar percentage.

The specific preparation method was as follows:

(a) The mRNA encoding p35 and p40 subunits of IL-12 polypeptide, the mRNA encoding IL-7 polypeptide, and the mRNA encoding IFN-$\alpha$ polypeptide at a mass ratio of 1:1:1 were dissolved in a citrate buffer at pH 4, and the concentration was adjusted to 0.1 mg/ml to obtain an aqueous phase.

(b) Dlin-MC3-DMA, DOPG, cholesterol, and PEG-DMG were dissolved in absolute ethanol according to the formula amount, and the concentration of the lipid component in the organic phase was adjusted to 6 mg/mL to obtain an organic phase.

(c) The aqueous phase of step (a) and the organic phase of step (b) were mixed at a volume ratio of 1:3 using a microfluidic device at a flow rate of 12 mL/min. The mixed solution was immediately diluted 100 times with PBS at pH 7.4, and tangential flow filtration (TFF) was used to remove the ethanol component in the solution. The solution was then concentrated until the concentration of mRNA in the system was 100 $\mu$g/ml, to obtain a lipid nanoparticle comprising RNA encoding IL-12 polypeptide, RNA encoding IL-7 polypeptide, and RNA encoding IFN-$\alpha$ polypeptide.

**Example 8**

This Example provides a method for preparing a lipid nanoparticle that independently encapsulates mRNA encoding three polypeptides, namely IL-12 p35 and p40 subunits, IL-7 polypeptide and IFN-α polypeptide. The lipid component of the lipid nanoparticle comprises Dlin-MC3-DMA 50%, DOPG 10%, cholesterol 38.5% and PEG-DMG 1.5% in molar percentage.

The specific preparation method was as follows:

(1) a lipid nanoparticle of RNA encoding IL-12 polypeptide

(a) The mRNA encoding p35 and p40 subunits of IL-12 polypeptide was dissolved in a citrate buffer at pH 4, and the concentration was adjusted to 0.1 mg/ml to obtain an aqueous phase.

(b) Dlin-MC3-DMA, DOPG, cholesterol, and PEG-DMG were dissolved in absolute ethanol according to the formula amount, and the concentration of the lipid component in the organic phase was adjusted to 6 mg/mL to obtain an organic phase.

(c) The aqueous phase of step (a) and the organic phase of step (b) were mixed at a volume ratio of 1:3 using a microfluidic device at a flow rate of 12 mL/min. The mixed solution was immediately diluted 100 times with PBS at pH 7.4, and tangential flow filtration (TFF) was used to remove the ethanol component in the solution. The solution was then concentrated until the concentration of mRNA in the system was 100 μg/ml, to obtain a lipid nanoparticle containing RNA encoding IL-12 polypeptide.

(2) The lipid nanoparticle of RNA encoding IL-7 polypeptide and the lipid nanoparticle of RNA encoding IFN-α polypeptide were prepared according to step (1) of this Example;

(3) The lipid nanoparticles of RNAs encoding three polypeptides were mixed at a mass ratio of 1:1:1 to obtain a lipid nanoparticle comprising RNA encoding IL-12 polypeptide, RNA encoding IL-7 polypeptide, and RNA encoding IFN-α polypeptide.

**Example 9**

This Example provides a method for preparing a lipid nanoparticle that simultaneously encapsulates DNA plasmids encoding three polypeptides, namely IL-12 p35 and p40 subunits, IL-7 polypeptide and IFN-α polypeptide. The DNAs encoding three polypeptides were integrated into three different plasmids. The lipid component of the lipid nanoparticle comprises Dlin-MC3-DMA 50%, DOPG 10%, cholesterol 38.5% and PEG-DMG 1.5% in molar percentage.

The specific preparation method was as follows:

(a) A DNA plasmid encoding p35 and p40 subunits of IL-12 polypeptide, a DNA plasmid encoding IL-7 polypeptide, and a DNA plasmid encoding IFN-α polypeptide at a mass ratio of 1:1:1 were dissolved in a citrate buffer at pH 4, and the concentration was adjusted to 0.1 mg/ml to obtain an aqueous phase.

(b) Dlin-MC3-DMA, DOPG, cholesterol, and PEG-DMG were dissolved in absolute ethanol according to the formula amount, and the concentration of the lipid component in the organic phase was adjusted to 6 mg/mL to obtain an organic phase.

(c) The aqueous phase of step (a) and the organic phase of step (b) were mixed at a volume ratio of 1:3 using a microfluidic device at a flow rate of 12 mL/min. The mixed solution was immediately diluted 100 times with PBS at pH 7.4, and tangential flow filtration (TFF) was used to remove the ethanol component in the solution. The solution was then concentrated until the concentration of DNA in the system was 100 μg/ml, to obtain the lipid nanoparticle comprising a DNA plasmid encoding IL-12 polypeptide, a DNA plasmid encoding an IL-7 polypeptide, and a DNA plasmid encoding IFN-α polypeptide.

**Example 10**

This Example provides a therapeutic fusion nucleic acid molecule.

Structure of the fusion RNA molecule: the fusion RNA molecule comprises IL-12 and IL-7 coding region, and additionally comprises (but is not limited to) untranslated region (UTR, such as 5'UTR or 3'UTR), 5' cap region, polyA tail region, starting region, termination region, signal sequence region, linker sequence and combinations thereof.

The protein cleavage signal encoded by the linker sequence comprises at least one protein cleavage site. The encoded protein cleavage signal may include, but is not limited to, protein precursor convertase (or hormone precursor convertase), thrombin and/or factor Xa protein cleavage signal, for example using the Furin cleavage site (FCS) (Ref. US7374930B2). One benefit of choosing FCS is that Furin is widely distributed in most cell types, and the fusion RNA of the present disclosure can effectively express an active polypeptide in almost any type of cell in the body.

The DNA sequence of the Furin cleavage site is CGTCAACGTCGT (SEQ ID NO. 30); the RNA sequence of the Furin cleavage site in the fusion RNA is CGUCAACGUCGU (SEQ ID NO. 31).

The structure of a fusion RNA including parts encoding IL-12 and IL-7 polypeptides is taken as an illustrative example: 5 cap' - 5' UTR - starting region - RNA encoding IL-12 - linker -RNA encoding IL -7 - 3' UTR - termination region - 3' polyA tail.

The linker can be, for example, a cleavable linker or a protease-sensitive linker. The linker is selected from the group consisting of cleavable linker F2A linker (with the amino acid sequence GSGVKQTLNFDLLKLAGDVESNPGP, SEQ ID NO. 32), P2A linker (with the amino acid sequence GSGATNFSLLKQAGDVEENPGP, SEQ ID NO. 33), T2A linker (with the amino acid sequence GSGEGRGSLLTCGDVEENPGP, SEQ ID NO. 34), E2A linker (e.g., having the amino acid sequence GSGQCTNYALLKLAGDVESNPGP, SEQ ID NO. 35) and combinations thereof.

A self-cleaving peptide may be, but is not limited to, 2A peptide. 2A peptides known in the art include, for example, foot-and-mouth disease virus (FMDV) 2A peptide, equine rhinitis A virus 2A peptide, *Thosea asigna* virus 2A peptide, and porcine teschovirus-1 2A peptide. Several viruses use the 2A peptide to produce two proteins from one transcript through ribosome hopping, such that the normal peptide bond is weakened at the 2A peptide sequence, resulting in the production of two discontinuous proteins from one translation event. The polynucleotide sequence encoding the 2A peptide includes, but is not limited to, the following sequence (the polynucleotide sequence of the 2A peptide can be modified or codon-optimized by the method described herein and/or known in the art):

(1)GGAAGCGGAGCUACUAACUUCAGCCUGCUGAAGCAGGCUGGAGA

CGUGGAGGAGAACCCUGGACCU (SEQ ID NO. 36);

or

(2)UCCGGACUCAGAUCCGGGGAUCUCAAAAUUGUCGCUCCUGUCAAA

CAAACUCUUAACUUUGAUUUACUCAAACUGGCTGGGGAUGUAGAAAGCA

AUCCAGGTCCACUC (SEQ ID NO. 37).

The structure of the therapeutic fusion nucleic acid molecule in the following Examples is: 5' cap - 5' UTR - starting region - RNA encoding IL-12 - linker - RNA encoding IL-7 - 3' UTR - termination region - 3' polyA tail; wherein the 5' cap region is m7Gppp (5') (2'-OMeA) pG (7-methylguanosine triphosphate-2-methoxymonophosphate guanosine cap analog; CAP1-GAG); the 5' UTR sequence is set forth in SEQ ID NO. 38; the starting region sequence is the promoter sequence, specifically AUG; the RNA encoding IL-12 is set forth in SEQ ID NO. 19; the linker RNA is set forth in SEQ ID NO. 31; the RNA encoding IL- 7 is set forth in SEQ ID NO. 22; the 3' UTR sequence is set forth in SEQ ID NO. 10; the termination region sequence is a terminator sequence, specifically UAA; the poly-A tail region is also called poly(A) and is 100A in length.

In the fusion RNA molecule, the nucleic acid fragment (B) (i.e., the RNA encoding IL-12) can be close to the 5' end, as shown above; it can also be close to the 3' end, and the specific structure is: 5' cap - 5' UTR - starting region - RNA encoding IL-7 - linker - RNA encoding IL-12 - 3' UTR - termination region - 3' polyA tail.

## Example 11

This Example provides therapeutic fusion nucleic acid molecules. The only difference from Example 10 is the structure of the fusion RNA molecule. The fusion RNA molecule in this example comprises parts encoding IL-12, IL-7 and IFN-α polypeptides, and additionally comprises, but is not limited to, the untranslated region (UTR, such as 5' UTR or 3' UTR), 5' cap region, poly A tail region, starting region, termination region, signal sequence region, linker sequence and combinations thereof.

The structure of the therapeutic fusion nucleic acid molecule is 5' cap - 5' UTR - starting region - RNA encoding IL-12 - linker - RNA encoding IL-7 - linker - RNA encoding IFN-α - 3' UTR - termination region - 3' polyA tail; wherein the 5' cap region is m7Gppp (5') (2' - OMeA) pG (7-methylguanosine triphosphate-2-methoxy guanosine monophosphate cap analog; CAP1-GAG); the 5' UTR sequence is set forth in SEQ ID NO. 38; the starting region sequence is the promoter sequence, specifically AUG; the RNA encoding IL-12 is set forth in SEQ ID NO. 19; the linker RNA is set forth in SEQ ID NO. 31; the RNA encoding IL- 7 is set forth in SEQ ID NO. 22; the RNA encoding IFN- α is set forth in SEQ ID NO. 26; 3' UTR sequence is set forth in SEQ ID NO. 10; the terminator sequence is a terminator sequence, specifically UAA;

the poly-A tail region is also called poly(A) and is 100A in length.

The relative order of the nucleic acid fragment (A), the nucleic acid fragment (B) and the nucleic acid fragment (C) in the fusion RNA molecule can be replaced, for example, it can be 5' cap - 5' UTR - starting region - RNA encoding IL -12 - linker - RNA encoding IFN-α - linker - RNA encoding IL-7 - 3' UTR - termination region - 3' polyA tail;

5' cap - 5' UTR - starting region - RNA encoding IL-12 - linker - RNA encoding IL-7 - linker - RNA encoding IFN- α - 3' UTR - termination region - 3' polyA tail;
5' cap - 5' UTR - starting region - RNA encoding IL-7 - linker - RNA encoding IFN-α - linker - RNA encoding IL-12 - 3' UTR - - termination region - 3' polyA tail;
5' cap - 5' UTR - starting region - RNA encoding IL-7 - linker - RNA encoding IL-12 - linker -RNA encoding IFN-α - 3' UTR - termination region - 3' polyA tail; 5' cap - 5' UTR - starting region - RNA encoding IFN-α - linker - RNA encoding IL-7 - linker - RNA encoding IL-12 - 3' UTR - termination region - 3' polyA tail;
5' cap - 5' UTR - starting region - RNA encoding IL-7 - linker - RNA encoding IL-12 - linker - RNA encoding IFN-α - 3'UTR - termination region - 3' polyA tail;
5' cap - 5'UTR - starting region - RNA encoding IFN-α - linker - RNA encoding IL-12 - linker - RNA encoding IL-7 - 3'UTR - termination region - 3'polyA tail.

## Example 12

This Example provides an LNP preparation of a therapeutic fusion nucleic acid molecule, and the preparation method was as follows: the fusion nucleic acid molecule mRNA encodes IL-12 p35 and p40 subunits and IL-7 polypeptide. The structure of the fusion nucleic acid molecule mRNA is as described in Example 10. The lipid component of the lipid nanoparticle comprises Dlin-MC3-DMA 50%, DOPG 10%, cholesterol 38.5% and PEG-DMG 1.5% in molar percentage.

The specific preparation method was as follows:

(a) The therapeutic fusion nucleic acid molecule mRNA was dissolved in a citrate buffer at pH 4 and the concentration was adjusted to 0.1 mg/ml to obtain an aqueous phase.
(b) Dlin-MC3-DMA, DOPG, cholesterol, and PEG-DMG were dissolved in absolute ethanol according to the formula amount, and the concentration of the lipid component in the organic phase was adjusted to 6 mg/mL to obtain an organic phase.
(c) The aqueous phase of step (a) and the organic phase of step (b) were mixed at a volume ratio of 1:3 using a microfluidic device at a flow rate of 12 mL/min. The mixed solution was immediately diluted 100 times with PBS at pH 7.4, and tangential flow filtration (TFF) was used to remove the ethanol component in the solution. The solution was then concentrated until the concentration of mRNA in the system was 100 μg/ml, to obtain the lipid nanoparticle containing the therapeutic fusion nucleic acid molecule mRNA.

## Example 13

This Example provides an LNP preparation of a therapeutic fusion nucleic acid molecule. The only difference from Example 12 is that the fusion nucleic acid molecule mRNA encodes IL-12 p35 and p40 subunits, IL-7 polypeptide and IFN-α polypeptide.

## Example 14

The lipid nanoparticle containing a therapeutic fusion nucleic acid molecule mRNA prepared in Example 13 was combined with the lipid nanoparticle containing the DNA plasmid encoding IL-12 polypeptide, the DNA plasmid encoding IL-7 polypeptide, and the DNA plasmid encoding IFN-α polypeptide prepared in Example 9, to obtain a second therapeutic nucleic acid molecule composition.

## Example 15

This Example provides a method for preparing a lipid nanoparticle that independently encapsulates mRNA encoding two polypeptides, namely IL-12 p35 and p40 subunits and IL-7 polypeptide. The lipid component of the lipid nanoparticle comprises Dlin-MC3-DMA 50%, DOPG 10%, cholesterol 38.5% and PEG-DMG 1.5% in molar percentage.

The specific preparation method was as follows:

(1) a lipid nanoparticle of RNA encoding IL-12 polypeptide

(a) The mRNA encoding p35 and p40 subunits of IL-12 polypeptide was dissolved in a citrate buffer at pH 4, and the concentration was adjusted to 0.1 mg/ml to obtain an aqueous phase.

(b) Dlin-MC3-DMA, DOPG, cholesterol, and PEG-DMG was dissolved in absolute ethanol according to the formula amount, and the concentration of the lipid component in the organic phase was adjusted to 6 mg/mL to obtain an organic phase.

(c) The aqueous phase of step (a) and the organic phase of step (b) were mixed at a volume ratio of 1:3 using a microfluidic device at a flow rate of 12 mL/min. The mixed solution was immediately diluted 100 times with PBS at pH 7.4, and tangential flow filtration (TFF) was used to remove the ethanol component in the solution. The solution was then concentrated until the concentration of mRNA in the system was 100 $\mu$g/ml, to obtain a lipid nanoparticle containing RNA encoding IL-12 polypeptide.

(2) The lipid nanoparticle of RNA encoding IL-7 polypeptide was prepared according to step (1) of this Example;

(3) The lipid nanoparticles of RNAs encoding two polypeptides were mixed at a mass ratio of 1:1:1 to obtain a lipid nanoparticle comprising RNA encoding IL-12 polypeptide and RNA encoding IL-7 polypeptide.

**Example 16**

This Example provides a method for preparing a lipid nanoparticle that simultaneously encapsulates mRNA encoding two polypeptides, namely IL-12 p35 and p40 subunits and IL-7 polypeptide. The lipid component of the lipid nanoparticle comprisees Dlin-MC3-DMA 50%, DOPG 10%, cholesterol 38.5% and PEG-DMG 1.5% in molar percentage.

The specific preparation method was as follows:

(a) The mRNA encoding p35 and p40 subunits of IL-12 polypeptide and the mRNA encoding IL-7 polypeptide at a mass ratio of 1:1 were dissolved in a citrate buffer at pH 4, and the concentration was adjusted to 0.1 mg/ml to obtain the aqueous phase.

(b) Dlin-MC3-DMA, DOPG, cholesterol, and PEG-DMG were dissolved in absolute ethanol according to the formula amount, and the concentration of the lipid component in the organic phase was adjust to 6 mg/mL to obtain an organic phase.

(c) The aqueous phase of step (a) and the organic phase of step (b) were mixed at a volume ratio of 1:3 using a microfluidic device at a flow rate of 12 mL/min. The mixed solution was immediately diluted 100 times with PBS at pH 7.4, and tangential flow filtration (TFF) was used to remove the ethanol component in the solution. The solution was then concentrated until the concentration of mRNA in the system was 100 $\mu$g/ml to obtain a lipid nanoparticle comprising RNA encoding IL-12 polypeptide and RNA encoding IL-7 polypeptide.

**Example 17**

This Example provides a method for preparing a lipid nanoparticle that simultaneously encapsulates DNA encoding two polypeptides, namely IL-12 p35 and p40 subunits and IL-7 polypeptide. The DNAs encoding the two polypeptides were integrated into two different plasmids. The lipid component of the lipid nanoparticle comprises Dlin-MC3-DMA 50%, DOPG 10%, cholesterol 38.5% and PEG-DMG 1.5% in molar percentage.

The specific preparation method was as follows:

(a) A DNA plasmid encoding p35 and p40 subunits of IL-12 polypeptide and a DNA plasmid encoding IL-7 polypeptide at a mass ratio of 1:1 were dissolved in a citrate buffer at pH 4, and the concentration was adjusted to 0.1 mg/ml to obtain an aqueous phase.

(b) Dlin-MC3-DMA, DOPG, cholesterol, and PEG-DMG were dissolved in absolute ethanol according to the formula amount, and the concentration of the lipid component in the organic phase was adjusted to 6 mg/mL to obtain an organic phase.

(c) The aqueous phase of step (a) and the organic phase of step (b) were mixed at a volume ratio of 1:3 using a microfluidic device at a flow rate of 12 mL/min. The mixed solution was immediately diluted 100 times with PBS at pH 7.4, and tangential flow filtration (TFF) was used to remove the ethanol component in the solution. The solution was then concentrated until the concentration of DNA in the system was 100 $\mu$g/ml, to obtain the lipid nanoparticle comprising a DNA plasmid encoding IL-12 polypeptide and a DNA plasmid encoding an IL-7 polypeptide.

**Example 18**

This Example examined the impact of mRNA combinations encoding different polypeptides on the tumor inhibition rate. Each combination comprises mRNA encoding two polypeptides, wherein the mRNA mixtures of groups F1 to F5 were prepared according to the preparation method in Example 15.

Six groups of experiments were conducted using the above mouse CT26 tumor model as the experimental model, with the number of mice in each group being 6:

Group F1 was administered with a mixture of luciferase mRNA, and the dosage was 0.06 mpk;

Group F2 was administered with a mixture of mRNA encoding p35 and p40 subunits of murine IL-12 (the nucleotide sequence is set forth in SEQ ID NO. 14), and the dosage was 0.04mpk;

Group F3 was administered with a mixture consisting of mRNA encoding p35 and p40 subunits of murine IL-12 (the nucleotide sequence is set forth in SEQ ID NO. 14) and mRNA encoding murine IL-7(the nucleotide sequence is set forth in SEQ ID NO. 15), and the dosage was IL-12 0.02mpk + IL-70.02mpk;

Group F4 was administered with a mixture consisting of mRNA encoding p35 and p40 subunits of murine IL-12 (the nucleotide sequence is set forth in SEQ ID NO. 14) and mRNA encoding murine IL15 (the nucleotide sequence is set forth in SEQ ID NO. 11), and the dosage was IL-12 0.02 mpk + IL15 0. 02 mpk;

Group F5 was administered with a mixture consisting of mRNA encoding p35 and p40 subunits of murine IL-12 (the nucleotide sequence is set forth in SEQ ID NO. 14) and mRNA encoding murine IL2 (the nucleotide sequence is set forth in SEQ ID NO. 12), and the dosage was IL-12 0.02 mpk + IL2 0. 02 mpk;

Group F6 was administered PD-1 antibody, and the dosage was 10 mpk.

The administration frequency of the mRNA mixture was once a week, and the administration period was 3 weeks. The administration method of the 6 groups of mice was all *in situ* injection. The 21$^{st}$ day after grouping was set as the endpoint of the experiment and the impact on tumor was tested for the six groups of experiments.

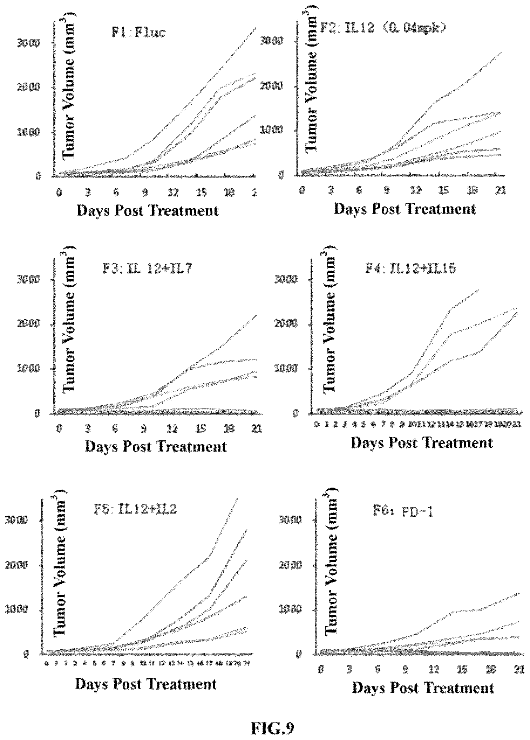

Verified by the above calculation method, at the endpoint of the experiment, the relative tumor inhibition rate ($TGI_{TV}$) of PD-1 antibody (F6 group) was 76.22%; the relative tumor inhibition rate ($TGI_{TV}$) of IL-12 (F2 group) was 32.66%; the relative tumor inhibition rate ($TGI_{TV}$) of the mixture of IL-12+IL-7(F3 group) was 54.38%; the relative tumor inhibition rate ($TGI_{TV}$) of the mixture of IL-12+IL15 (F4 group) was 49.70%; and the relative tumor inhibition rate of the mixture of IL-12+IL2 ($TGI_{TV}$) (F5 group) was -3.36%. The results of the changes in tumor volume with the injection days in each experimental group are shown in Figure 8, and the changes in tumor volume of each mouse in each experimental group are shown in Figure 9.

**Example 19**

This Example is provided in order to examine whether the mouse with tumor regression after injection of an LNP preparation containing the two components of IL-12 + IL-7 can produce immune memory. In this Example, mice with tumor regression were selected, and mice were re-inoculated with colon cancer cells CT26 ($5\times10^5/100\ \mu L$/mouse) 47 days after the first administration (IL-12+IL-7 0.04 mpk each). Following tumor cell re-inoculation, tumor size was observed on day 61, 68, 71, 75 and 77 after the first dose, and the results are shown in the figure.

G1 represented 6 mice that were never injected with the two components of IL-12 + IL-7; G2 represented 2 mice that were injected with the two components of murine IL-12 (the nucleotide sequence is set forth in SEQ ID NO. 14) + murine IL-7 (the nucleotide sequence is set forth in SEQ ID NO. 13) and showed tumor regression.

The results showed: 1) The tumor volume of G1 mice that were not injected with the two components of IL-12+IL-7 at the endpoint of the experiment reached 462.44 + 86.92 mm$^3$;

2) G2 mice that had tumor regression and were re-inoculated with tumor cells still showed no tumor growth at the endpoint of the trial.

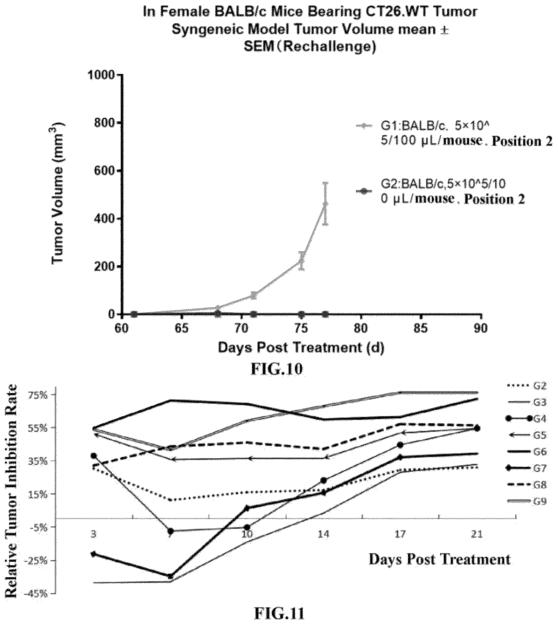

From the above results, it can be concluded that upon injection of the two components of IL-12 + IL-7, the effect of tumor regression can be achieved, and after the tumor subsided, re-inoculation of tumor cells can also inhibit tumor growth, indicating that long-term immune memory has been established in animals after injection of the mixture of IL-12+IL-7. The graph illustrating the changes in tumor volume of each experimental group is shown in Figure 10.

**Example 20**

This Example examined the effect of mRNA combinations encoding different murine polypeptides on the tumor inhibition rate. The mRNA mixtures of groups G1 to G8 were prepared according to the preparation method in Example 8 or Example 15.

Nine groups of experiments were conducted using the above mouse CT26 tumor model as the experimental model, with the number of mice in each group being 6.

Group G1 was administered with luciferase mRNA, and the dosage was 0.06mpk;

Group G2 was administered with mRNA encoding p35 and p40 subunits of murine IL-12 (the nucleotide sequence is set forth in SEQ ID NO. 14), and the dosage was 0.02mpk;

Group G3 was administered with mRNA encoding p35 and p40 subunits of murine IL-12 (the nucleotide sequence is set forth in SEQ ID NO. 14), and the dosage was 0.04mpk;

Group G4 was administered with a mixture of mRNA encoding p35 and p40 subunits of murine IL-12 (the nucleotide sequence is set forth in SEQ ID NO. 14), and the dosage was 0.08mpk;

Group G5 was administered with a mixture consisting of mRNA encoding p35 and p40 subunits of murine IL-12 (the nucleotide sequence is set forth in SEQ ID NO. 14) and mRNA encoding murine IL-7(the nucleotide sequence is set forth in SEQ ID NO. 15 (shown), and the dosage was IL-12 0.02mpk + IL-70.02mpk;

Group G6 was administered with a mixture consisting of mRNA encoding p35 and p40 subunits of murine IL-12 (the nucleotide sequence is set forth in SEQ ID NO. 14), mRNA encoding murine IL-7(the nucleotide sequence is set forth in SEQ ID NO. 15) and mRNA encoding murine IFN-$\alpha$ (the nucleotide sequence is set forth in SEQ ID NO. 13), and the dosage was IL-12 0.02mpk + IL-70.02mpk + IFN -$\alpha$ 0.02mpk;

Group G7 was administered with a mixture consisting of mRNA encoding p35 and p40 subunits of murine IL-12 (the nucleotide sequence is set forth in SEQ ID NO. 14), mRNA encoding murine IL-7(the nucleotide sequence is set forth in SEQ ID NO. 15) and mRNA encoding murine IL15 (the nucleotide sequence is set forth in SEQ ID NO. 11), and the dosage was IL-12 0.02mpk + IL-70.02mpk + IL15 0.02mpk;

Group G8 was administered with a mixture consisting of mRNA encoding p35 and p40 subunits of murine IL-12 (the nucleotide sequence is set forth in SEQ ID NO. 14), mRNA encoding murine IL-7(the nucleotide sequence is set forth in SEQ ID NO. 15) and mRNA encoding murine IL2 (the nucleotide sequence is set forth in SEQ ID NO. 12), and the dosage was IL-12 0.02mpk + IL-70.02mpk + IL2 0.02mpk;

Group G9 was administered with PD-1 antibody, and the dosage was 10 mpk.

The administration frequency of the mRNA mixture was once a week, and the administration period was 3 weeks. The administration method of the 9 groups of mice was all *in situ* injection. The 21st day after grouping was set as the endpoint of the experiment and the impact on tumor was tested for the nine groups of experiments.

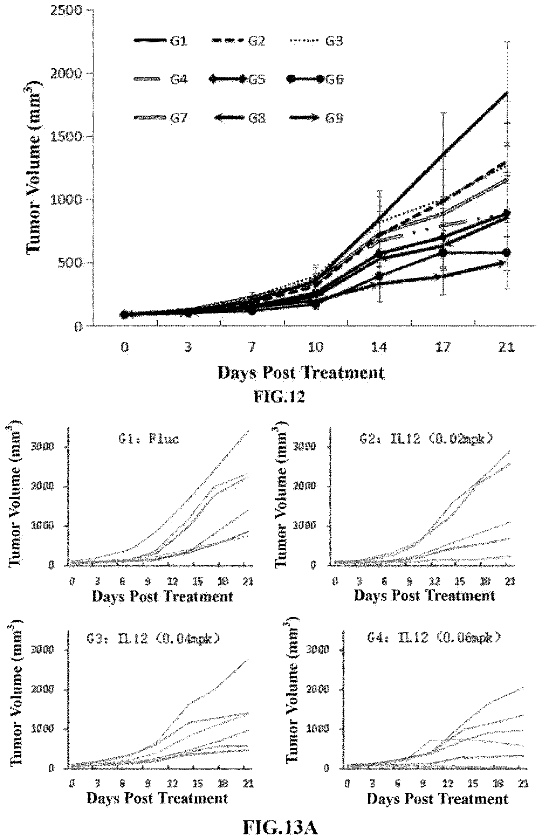

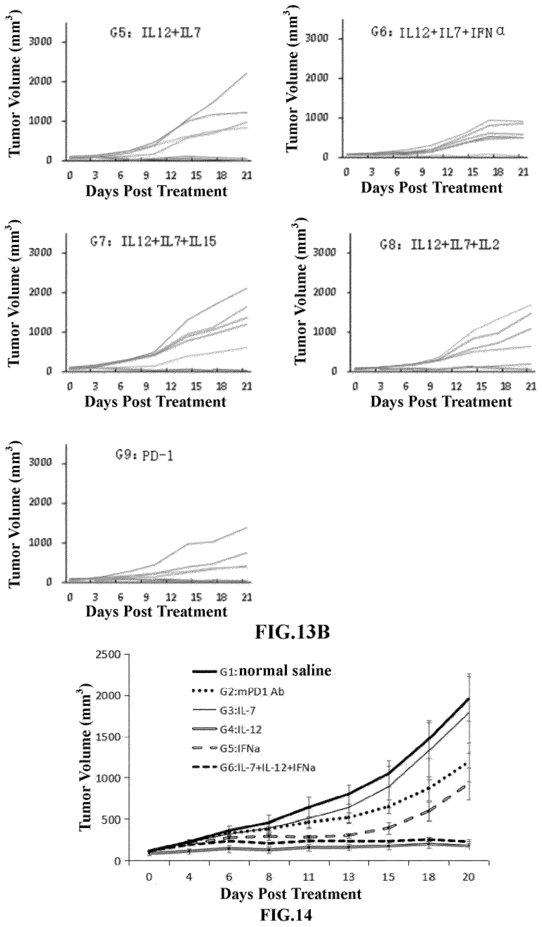

Verified by the above calculation method, at the endpoint of the experiment, the relative tumor inhibition rate ($TGI_{TV}$) of PD-1 antibody (G9 group) was 76.22%; the relative tumor inhibition rate of the mixture of IL-12+IL7+IFN-$\alpha$ (G6 group) ($TGI_{TV}$) was 72.27%; the relative tumor inhibition rate ($TGI_{TV}$) of the mixture of IL-12+IL-7(G5 group), G4 group, and the mixture of IL-12+IL7+IL15 (G8 group) were 54.38%, 54.41%, and 56.22% respectively; and the relative tumor inhibition rates ($TGI_{TV}$) of other groups were less than 40%. The curves of relative tumor inhibition rate ($TGI_{TV}$) of each experimental group are shown in Figure 11; the results of changes in the tumor volume with injection days in each experimental group are shown in Figure 12. The changes in tumor volume of each mouse in each experimental group are shown in Figure 13 A and Figure 13 B.

**Example 21**

This Example examined the effect of a combination of mRNAs encoding different murine polypeptides on the tumor inhibition rate. The mRNA mixtures of groups F3 to F6 were prepared according to the preparation method in Example 15.

Six groups of experiments were conducted using the above mouse CT26 tumor model as the experimental model, with the number of mice in each group being 6:

Group F1 was administered with normal saline;

Group F2 was administered with mPD1 Ab, and the dosage was 10 mpk;

Group F3 was administered with mRNA encoding murine IL-7(the nucleotide sequence is set forth in SEQ ID NO. 15), and the dosage was IL-7 1.2mpk;

Group F4 was administered with mRNA encoding p35 and p40 subunits of murine IL-12 (the nucleotide sequence is set forth in SEQ ID NO. 14), and the dosage was 1.2mpk;

Group F5 was administered with mRNA encoding murine IFN-$\alpha$ (the nucleotide sequence is set forth in SEQ ID NO. 13), and the dosage was 1.2mpk;

Group F6 was administered with a mixture consisting of mRNA encoding p35 and p40 subunits of murine IL-12 (the nucleotide sequence is set forth in SEQ ID NO. 14), mRNA encoding murine IL-7(the nucleotide sequence is set forth in SEQ ID NO. 15) and mRNA encoding murine IFN-$\alpha$ (the nucleotide sequence is set forth in SEQ ID NO. 13), and the dosage was IL-12 1.2mpk + IL-71.2mpk + IFN-$\alpha$ 1.2mpk.

The administration frequency of the mRNA mixture was once a week, and the administration period was 3 weeks. The administration method of the 6 groups of mice was all *in situ* injection. The 20th day after grouping was set as the endpoint of the experiment and the impact on tumor was tested for the six groups of experiments.

Verified by the above calculation method, at the endpoint of the experiment, the relative tumor inhibition rate (TGI$_{TV}$) of mPD1 Ab (F2 group) was 41.49%; the relative tumor inhibition rate (TGI$_{TV}$) of IL-7(F3 group) was 9.32%; the relative tumor inhibition rate (TGI$_{TV}$) of IL-12 (F4 group) was 95.01%; the relative tumor inhibition rate (TGI$_{TV}$) of IFN-$\alpha$ (F5 group) was 55.88%; and the relative tumor inhibition rate (TGI$_{TV}$) of the mixture of IL-12+IL-7+IFN-$\alpha$ (F6 group) was 94.78%. The results of changes in the tumor volume with injection days in each experimental group are shown in Figure 14.

## Example 22

This Example examined the impact of different dosages on the tumor inhibitory effect.

The above mouse MDA-MB-231 tumor model was used as the experimental model. On the 16th day after construction (14.8 weeks after immune reconstitution), 30 humanized tumor-bearing mice were selected and randomly divided into 7 groups according to tumor volume, with 6 mice in each group. The day of grouping was defined as D0, and administration started on the same day of grouping (D0). The remainder of the mice upon grouping was euthanized.

Group G1 dosage regimen: a mixture of luciferase mRNA, at a dosage of 0.06 mpk;

Group G2 dosage regimen: mRNA encoding human IL-12 p35 and p40 subunits (the nucleotide sequence is set forth in SEQ ID NO. 19), at a dosage of 0.04mpk;

Group G3 dosage regimen: a mixture consisting of mRNA encoding human IL-12 p35 and p40 subunits (the nucleotide sequence is set forth in SEQ ID NO. 19), mRNA encoding human IL-7 (nucleotide sequence is set forth in SEQ ID NO. 22) and mRNA encoding human IFN-$\alpha$ (the nucleotide sequence is set forth in SEQ ID NO. 26), at a weight ratio of 1:1:1 for the three RNAs in the mixture and at a dosage of 0.022mpk;

Group G4 dosage regimen: a mixture consisting of mRNA encoding human IL-12 p35 and p40 subunits (the nucleotide sequence is set forth in SEQ ID NO. 19), mRNA encoding human IL-7 (the nucleotide sequence is set forth in SEQ ID NO. 22) and mRNA encoding human IFN-$\alpha$ (the nucleotide sequence is set forth in SEQ ID NO. 26), at a weight ratio of 1:1:1 for the three RNAs in the mixture and at a dosage of 0.067mpk;

Group G5 dosage regimen: a mixture consisting of mRNA encoding human IL-12 p35 and p40 subunits (the nucleotide sequence is set forth in SEQ ID NO. 19), mRNA encoding human IL-7 (the nucleotide sequence is set forth in SEQ ID NO. 22) and mRNA encoding human IFN-$\alpha$ (the nucleotide sequence is set forth in SEQ ID NO. 26), at a weight ratio of 1:1:1 for the three RNAs in the mixture and at a dosage of 0.2mpk;

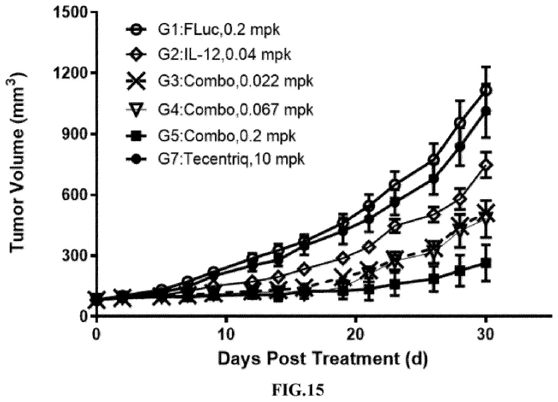

Group G6 dosing regimen: 10 mpk Tecentriq (positive drug). The changes in tumor volume for mice of G1 to G6 are shown in Figure 15.

According to the statistical analysis of tumor volume data at the time of drug withdrawal, compared with the control group: IL-12 in G2 Group (TGI$_{TV}$= 35.67%) can significantly inhibit tumor growth (P<0.05*); the low dose G3 Group (TGI$_{TV}$ = 58.50%), G4 Group (TGI$_{TV}$ = 61.39%) and G5 Group (TGI$_{TV}$ = 82.34%) can significantly inhibit tumor growth (P<0.05 *).

The pharmacodynamic effects of different doses of the subject mRNA three-component mixture and the positive drug Tecentriq in the tumor model of huHSC-NCG-hIL15 mouse subcutaneously loaded with the MDA-MB-231 breast cancer were evaluated in this experiment. The above experimental data indicated that under the current test system, the subject mRNA three-component mixture at low, medium and high doses all showed significant tumor inhibitory effects in terms of tumor volume compared with the control group G1.

## Example 23

Experimental method: This example examined the effects of a mixture of polypeptide-encoding mRNA combinations prepared by different processes on the tumor inhibition rate. The mRNA mixture of T1 group was prepared according to the preparation method in Example 12; the mRNA mixture of T2 group was prepared according to the preparation method in Example 16; the mRNA mixtures of T3 group and T4 group were prepared according to the preparation method in Example 15.

Four groups of experiments were conducted using the above mouse MDA-MB-231 tumor model as the experimental model, with the number of mice in each group being 6.

Group 2.1 (T1) represented mRNA encoding human IL-12 and humanized IL-7 fusion protein (the nucleotide sequences of the open reading frame are set forth in SEQ ID NO. 19 and SEQ ID NO. 22), and the dosage was 0.04 mpk;

Group 2.2 (T2) represented a mixture consisting of mRNA encoding human IL-12 p35 and p40 subunits (the nucleotide sequence is set forth in SEQ ID NO. 19) and mRNA encoding human IL-7 (the nucleotide sequence is set forth in SEQ ID NO. 22), and the dosage was IL-12 mRNA 0.02mpk and IL-7 mRNA 0.02mpk;

Group 2.3 (T3) represented a mixture consisting of mRNA encoding human IL-12 p35 and p40 subunits (the nucleotide sequence is set forth in SEQ ID NO. 19) and mRNA encoding human IL-7 (the nucleotide sequence is set forth in SEQ ID NO. 22), and the dosage was IL-12 0.02mpk + IL-7 0.02mpk;

Group 2.4 (T4) was administered with luciferase mRNA, and the dosage was 0.48mpk.

The administration frequency of the mRNA mixture was once a week, and the administration period was 3 weeks. The administration method of the 6 groups of mice was all *in situ* injection. The 33rd day after grouping was set as the endpoint of the experiment and the impact on tumor was tested for the six groups of experiments.

Verified by the above calculation method, at the endpoint of the experiment, the relative tumor inhibition rate ($TGI_{TV}$) of T1 group was 53.21%; the relative tumor inhibition rate ($TGI_{TV}$) of T2 group was 54.78%; and the relative tumor inhibition rate ($TGI_{TV}$) of T3 group was 54.34%.

**Example 24**

Experimental method: This Example examined the impact of mRNA combinations encoding different polypeptides on the tumor inhibition rate. The mRNA mixtures of Group P2, Group P3 and Group P4 were prepared according to the preparation method in Example 15.

Four groups of experiments were conducted using the above mouse MDA-MB-231 tumor model as the experimental model, with the number of mice in each group being 6.

Group P1 was administered with a mixture of luciferase mRNA, and the dosage was 0.48mpk.

Group P2 was administered with a mixture consisting of mRNA encoding human IL-12 p35 and p40 subunits (the nucleotide sequence is set forth in SEQ ID NO. 19) and mRNA encoding human IL-7 (the nucleotide sequence is set forth in SEQ ID NO. 22), and the dosage was IL-12 0.02mpk + IL-7 0.02mpk.

Group P3 was administered with a mixture consisting of mRNA encoding human IL-12 p35 and p40 subunits (the nucleotide sequence is set forth in SEQ ID NO. 19) and mRNA encoding human IL-7 (the nucleotide sequence is set forth in SEQ ID NO. 19) SEQ ID NO. 23), and the dosage was IL-12 0.02mpk + IL-7 0.02mpk.

Group P4 was administered with a mixture consisting of mRNA encoding human IL-12 p35 and p40 subunits (the nucleotide sequence is set forth in SEQ ID NO. 19) and mRNA encoding human IL-7 (the nucleotide sequence is set forth in SEQ ID NO. 24), and the dosage was IL-12 0.024mpk + IL-7 0.024mpk.

The administration frequency of the mRNA mixture was once a week, and the administration period was 3 weeks. The administration method of the 5 groups of mice was all *in situ* injection. The 33rd day after grouping was set as the endpoint of the experiment and the impact on tumor was tested for the five groups of experiments.

Verified by the above calculation method, at the endpoint of the experiment, the relative tumor inhibition rate ($TGI_{TV}$) of Group P2 was 54.38%; the relative tumor inhibition rate ($TGI_{TV}$) of Group P3 was 50.23%; and the relative tumor inhibition rate ($TGI_{TV}$) of Group P4 was 55.01%.

In the present disclosure, when any one of the three mRNA sequences encoding IL-7 polypeptide was employed for the ORF of mRNA encoding IL-7 polypeptide in the case of the mixture of IL-12 + IL-7 and the dosage was adjusted according to the IL-7 mRNA expression level of each sequence; such effects as tumor regression, relative tumor inhibition rate ($TGI_{TV}$) were not affected. When any one of the three sequences of IL-12 mRNA (as set forth in SEQ ID NOs. 19-21), was employed for the ORF of mRNA encoding IL-12 in the case of the mixture of IL-12 + IL-7 and when the dosage was adjusted according to the IL-12 expression level of each sequence, such effects as tumor regression, relative tumor inhibition rate ($TGI_{TV}$) were not affected.

Example 25

This Example provides a pharmaceutical composition comprising the following components:

| serial number | Nucleic acid molecule fragment (A) | Nucleic acid molecule fragment (B) | Nucleic acid molecule fragment (C) | protein drug |
|---|---|---|---|---|
| 54 | Encoding p35 and p40 subunits of interleukin IL-12 | Encoding IL 7 polypeptide | Encoding IFN-$\alpha$ polypeptide | anti-PD-1 antibody |
| 65 | Encoding p35 and p40 subunits of interleukin IL-12 | Encoding IL 7 polypeptide | Encoding IFN-$\alpha$ polypeptide | anti-PD-L1 antibody |

(continued)

| serial number | Nucleic acid molecule fragment (A) | Nucleic acid molecule fragment (B) | Nucleic acid molecule fragment (C) | protein drug |
|---|---|---|---|---|
| 76 | Encoding p35 and p40 subunits of interleukin IL-12 | Encoding IL 7 polypeptide | Encoding IFN-α polypeptide | anti-CTLA-4 antibody |
| 87 | Encoding p35 and p40 subunits of interleukin IL-12 | Encoding IL 7 polypeptide | / | anti-PD-1 antibody |
| 18 | Encoding p35 and p40 subunits of interleukin IL-12 | Encoding IL 7 polypeptide | / | anti-PD-L1 antibody |
| 19 | Encoding p35 and p40 subunits of interleukin IL-12 | Encoding IL 7 polypeptide | / | anti-CTLA-4 antibody |

Finally, it should be noted that the above embodiments are only intended to be illustrative of the technical solutions of the present disclosure, instead of limiting; although the present disclosure has been described in detail with reference to the foregoing embodiments, those of ordinary skill in the art should appreciate that, it is still possible to modify the technical solutions recited in the foregoing embodiments, or to equivalently replace some or all of the technical features; and these modifications or substitutions do not render the essence of the corresponding technical solutions deviating from the scope of the technical solutions of various embodiments of the present disclosure.

**Industrial applicability**

The nucleic acid molecule or nucleic acid molecule mixture provided by the present disclosure, wherein the main components are the nucleic acid molecule fragments encoding IL-7 protein and encoding p35 and p40 subunits of interleukin IL-12, can fully activate a variety of immune cell activities to achieve effective treatment of solid tumors. The present disclosure also provides a nucleic acid molecule containing the above nucleic acid molecule fragments in different combination forms. The combination forms include the fusion of different nucleic acid molecule fragments, and a composition formed by the different nucleic acid molecule fragments in free form. It has been verified that both types of the nucleic acid molecule fragments provided by the present disclosure, whether combined in free form or prepared into a fusion nucleic acid molecule, have shown effective therapeutic effects on solid tumors. The present disclosure further uses an aptamer for complexing with the above-mentioned nucleic acid molecule composition or fusion nucleic acid molecule to obtain a nucleic acid molecule drug, and provides a corresponding preparation method, in order to alleviate the current urgent need for drugs in the treatment of solid tumors.

**Claims**

1. A therapeutic nucleic acid molecule or a nucleic acid molecule mixture, **characterized in that** the nucleic acid molecule comprises: a nucleic acid molecule fragment (A) encoding an IL-7 protein; and a nucleic acid molecule fragment (B) encoding p35 and p40 subunits of interleukin IL-12.

2. The therapeutic nucleic acid molecule or nucleic acid molecule mixture according to claim 1, further comprising a nucleic acid molecule fragment (C), wherein the nucleic acid molecule fragment (C) comprises a nucleic acid fragment encoding at least one of the following proteins: IFN-α, IFN-β, IFN-γ, GM-CSF, IL15 and IL-2;

   preferably, the nucleic acid molecule fragment (C) encodes IFN-α;
   preferably, the amino acid sequence of IL-7 encoded by the nucleic acid molecule fragment (A) is set forth in SEQ ID NO. 42, or comprises an amino acid sequence having at least 80% identity to the amino acid sequence set forth in SEQ ID NO. 42;
   preferably, the nucleotide sequence of the nucleic acid molecule fragment (A) is selected from any one of SEQ ID NOs. 22-24 and SEQ ID NOs. 47-49; or selected from any one of the nucleotide sequences with at least 70% identity to the nucleotide sequences set forth in SEQ ID NO. 22-24s and SEQ ID NOs. 47-49;

preferably, the amino acid sequence of the p35 subunit of IL-12 encoded by the nucleic acid molecule fragment (B) is set forth in SEQ ID NO. 39, or comprises an amino acid sequence having at least 80% identity to the amino acid sequence set forth in SEQ ID NO. 39;

preferably, the amino acid sequence of the p40 subunit of IL-12 encoded by the nucleic acid molecule fragment (B) is set forth in SEQ ID NO. 40, or comprises an amino acid sequence having at least 80% identity to the amino acid sequence set forth in SEQ ID NO. 40;

preferably, the p35 subunit and p40 subunit of IL-12 encoded by the nucleic acid molecule fragment (B) are connected through a linker, wherein the amino acid sequence of the linker is set forth in SEQ ID NO. 41;

preferably, the nucleotide sequence of the nucleic acid molecule fragment (B) is selected from any one of SEQ ID NOs. 19-21 and SEQ ID NOs. 44-46; or selected from any one of the nucleotide sequences with at least 70% identity to the nucleotide sequences set forth in SEQ ID NOs. 19-21 and SEQ ID NOs. 44-46;

preferably, the amino acid sequence of the IFN-$\alpha$ polypeptide encoded by the nucleic acid molecule fragment (C) is set forth in SEQ ID NO. 43, or comprises an amino acid sequence having at least 80% identity to the nucleotide sequence set forth in SEQ ID NO. 43;

preferably, the nucleotide sequence of the nucleic acid molecule fragment (C) is selected from any one of SEQ ID NOs. 26-28 and SEQ ID NOs. 50-52, or selected from any one of the nucleotide sequences with at least 70% identity to the nucleotide sequence set forth in SEQ ID NOs. 26 -28 and SEQ ID NOs. 50-52.

3. The therapeutic nucleic acid molecule or nucleic acid molecule mixture according to claim 1 or 2, **characterized in that** the nucleic acid molecule comprises DNA molecule and/or RNA molecule;

preferably, the DNA molecule comprises stranded DNA molecule and/or circular DNA molecule;
preferably, the RNA molecule comprises mRNA or circular RNA;
preferably, the 5' end and/or 3' end of the nucleic acid molecule fragment has a modifying group;
preferably, the nucleic acid molecule fragment is an mRNA fragment, and the 5' end modifying group of the mRNA fragment is selected from ARCA, m7G(5')ppp(5')(2'OMeA)pG, m7G(5')ppp(5')(2'OMeG)pG, m7(3'OMeG)(5')ppp(5')(2'OMeG)pG, m7(3'OMeG)(5')ppp(5')(2'OMeA)pG, mCAP, dmCAP, tmCAP or dmCAP;
preferably, the 3' end protective modifying group of the mRNA fragment is poly(A), and the length of the poly(A) is 50 to 200, preferably 80 to 200;
preferably, the mRNA fragment further comprises a 5'UTR;
preferably, the length of the 5'UTR is preferably 10 to 200 nucleotides, preferably 15 to 100 nucleotides;
preferably, the 5'UTR comprises KOZAK sequence or DNAH2 5'UTR;
preferably, the nucleotide sequence of the KOZAK sequence is set forth in SEQ ID NO. 16;
preferably, the nucleotide sequence of DNAH2 5'UTR is set forth in SEQ ID NO. 38;
preferably, the mRNA fragment further comprises a 3'UTR;
preferably, the 3'UTR sequence is set forth in SEQ ID NOs. 1-10;
preferably, the mRNA sequentially comprises a 5' cap, 5' UTR, ORF, 3' UTR and 3' poly(A) tail from the 5' end to the 3' end.

4. A first therapeutic nucleic acid molecule composition, **characterized in that** the first therapeutic nucleic acid molecule composition comprises the nucleic acid molecule mixture according to any one of claims 1 to 3;
preferably, the mass ratio between individual free nucleic acid molecule fragments in the first therapeutic nucleic acid molecule composition is 10:1 to 1:10.

5. A therapeutic fusion nucleic acid molecule, **characterized in that** the therapeutic fusion nucleic acid molecule comprises the nucleic acid molecule according to any one of claims 1 to 3;

preferably, at least any two of the nucleic acid molecule fragments are connected through a linker;
preferably, the mass ratio between individual nucleic acid molecule fragments in the therapeutic fusion nucleic acid molecule is 10:1-1:10.

6. A second therapeutic nucleic acid molecule composition, **characterized in that** the second therapeutic nucleic acid molecule composition comprises a combination of the first therapeutic nucleic acid molecule composition according to claim 4 and the therapeutic fusion nucleic acid molecule according to claim 5;
preferably, the mass ratio between individual nucleic acid molecule fragments in the second therapeutic nucleic acid molecule composition is 10:1-1:10.

7. Use of the therapeutic nucleic acid molecule or nucleic acid molecule mixture according to any one of claims 1 to 3, the first therapeutic nucleic acid molecule composition according to claim 4, the therapeutic fusion nucleic acid molecule according to claim 5, or the second therapeutic nucleic acid molecule composition according to claim 6 for the preparation of anti-solid tumor drugs or for the evaluation of the efficacy of solid tumor drugs;

preferably, the solid tumor includes epithelial tumor, Hodgkin's lymphoma, non-Hodgkin's lymphoma, prostate tumor, ovarian tumor, renal cell tumor, gastrointestinal tumor, liver tumor, colorectal tumor, hemangioma, mesothelioma, pancreatic tumor, breast tumor, sarcoma, lung tumor, colon tumor, brain tumor, melanoma, small cell lung tumor, neuroblastoma, testicular tumor, carcinoid tumor, adenocarcinoma, glioma, sperm cell carcinoma, retinoblastoma or osteosarcoma.

8. A nucleic acid molecule drug for treating solid tumors, **characterized in that** the nucleic acid molecule drug comprises nucleic acid molecule component(s) and a carrier that encapsulates the nucleic acid molecule component(s);

the nucleic acid molecule component is selected from the therapeutic nucleic acid molecule or nucleic acid molecule mixture according to any one of claims 1 to 3, the first therapeutic nucleic acid molecule composition according to claim 4, the therapeutic fusion nucleic acid molecule according to claim 5, or the second therapeutic nucleic acid molecule composition according to claim 6;
preferably, the mass ratio between individual free nucleic acid molecule fragments is 10:1-1:10;
preferably, the mass ratio between individual nucleic acid molecule fragments in the fusion nucleic acid molecule is 10: 1-1: 10;
preferably, the carrier comprises a liposomal nanoparticle;
preferably, each of the free nucleic acid molecule fragments or the fusion nucleic acid molecule in the nucleic acid molecule component is independently encapsulated by the liposomal nanoparticle;
preferably, at least two of the free nucleic acid molecule fragments or the fusion nucleic acid molecules are independently encapsulated by the liposomal nanoparticle;
preferably, at least two of the free nucleic acid molecule fragments are co-encapsulated by the liposomal nanoparticle;
preferably, at least one of the free nucleic acid molecule fragments and the fusion nucleic acid molecule are co-encapsulated by the liposomal nanoparticle;
preferably, the liposomal nanoparticle comprises 20% to 50% cationic lipid, 20% to 50% DOPG, 5% to 20% cholesterol and 1% to 5% PEG-DMG in molar percentage;
preferably, the liposomal nanoparticle comprises 50% Dlin-MC3-DMA, 10% DOPG, 38.5% cholesterol and 1.5% PEG-DMG in molar percentage;
preferably, the nucleic acid molecule drug further comprises a therapeutic protein or a therapeutic chemical pharmaceutical component;
preferably, the therapeutic protein comprises atezolizumab.

9. A method for preparing the nucleic acid molecule drug according to claim 8, **characterized in that**: dissolving the free nucleic acid molecule fragment(s) and/or the fusion nucleic acid molecule in a buffer to obtain an aqueous phase, measuring individual components of the encapsulating carrier and dissolving said components in an organic solvent to obtain an organic phase, mixing the aqueous phase and the organic phase, and then removing the organic phase to obtain the nucleic acid molecule drug;

preferably, the volume ratio of the aqueous phase and the organic phase is 1:2-4, more preferably 1:3;
preferably, the buffer comprises citrate buffer or sodium acetate, more preferably citrate buffer;
preferably, the pH of the buffer is 3 to 7, more preferably 4;
preferably, the concentration of the free nucleic acid molecule fragment or fusion nucleic acid molecule in the aqueous phase is 0.05 mg/mL-0.5 mg/mL, more preferably 0.1 mg/mL;
preferably, the organic solvent is selected from C1 to C4 lower alcohols, more preferably anhydrous ethanol;
preferably, the concentration of the lipid component in the organic phase is 5 mg/mL-7 mg/mL, more preferably 6 mg/mL;
preferably, the aqueous phase and the organic phase are mixed by microfluidics, and the organic solvent is filtered using tangential flow;
preferably, the flow rate of the microfluidics is >3 ml/min, more preferably 12 mL/min;
preferably, the method further comprises a concentration step after mixing, wherein the concentration step allows the final concentration of the free nucleic acid molecule fragment(s) and/or the fusion nucleic acid molecule to be from 50 $\mu$g/mL to 200 $\mu$g/mL, more preferably 100 $\mu$g/mL.

10. A pharmaceutical composition, **characterized in that** the pharmaceutical composition comprises the nucleic acid molecule drug according to claim 8 or the nucleic acid molecule drug prepared by the method according to claim 9;

preferably, the pharmaceutical composition further comprises a protein drug selected from the group consisting of at least one of anti-PD-1 antibody, anti-PD-L1 antibody, anti-CTLA-4 antibody, anti-DC20 antibody, anti-Her2 antibody, anti-CD33 antibody, anti-CD52 antibody, anti-VEGFR antibody, anti-EGFR antibody, anti-RANKL antibody, anti-CD30 antibody, anti-VEGFR2 antibody, anti-GD2 antibody, anti-CD38 antibody, anti-CD22 antibody and anti-CD33 antibody;

preferably, the pharmaceutical composition further comprises at least one of atezolizumab, nivolumab, pembrolizumab, pidilizumab, durvalumab, avelumab and ipilimumab.

FIG.1

**Days Post Injection**

FIG.2

TGI=19.67%*

TGI=45.52%***

TGI=73.94%***

TGI=95.10%***

**Days Post Treatment (d)**

FIG.3

**FIG.4**

**FIG.5**

**FIG.6**

FIG.7

**Days Post Treatment**

FIG.8

**FIG.9**

In Female BALB/c Mice Bearing CT26.WT Tumor
Syngeneic Model Tumor Volume mean ±
SEM(Rechallenge)

FIG.10

FIG.11

FIG.12

FIG.13A

**FIG.13B**

**FIG.14**

FIG.15

## INTERNATIONAL SEARCH REPORT

International application No.

**PCT/CN2023/092440**

### A. CLASSIFICATION OF SUBJECT MATTER

A61K31/7088(2006.01)i; C12N15/62(2006.01)i; A61P35/00(2006.01)i

According to International Patent Classification (IPC) or to both national classification and IPC

### B. FIELDS SEARCHED

Minimum documentation searched (classification system followed by classification symbols)

IPC: A61K,C12N,A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pubmed, STN on web, genbank, NCBI, 中文专利序列检索系统, 珠海丽凡达生物技术有限公司, 彭育才, 治疗性核酸分子, therapeutic nucleic acid, IL-7, IL-12, P35, P40, IFN, 协同, synergy, 肿瘤, cancer, tumor, SEQ ID NOs: 19-24, 26-29, 39-42, 44-52

### C. DOCUMENTS CONSIDERED TO BE RELEVANT

| Category* | Citation of document, with indication, where appropriate, of the relevant passages | Relevant to claim No. |
|---|---|---|
| Y | US 2017136072 A1 (UNIVERSITY HEALTH NETWORK) 18 May 2017 (2017-05-18) claims 1, 13, description, paragraphs 15, 17 | 1-10, |
| Y | NAKAO, S. et al. "Intratumoral expression of IL-7 and IL-12 using an oncolytic virus increases systemic sensitivity to immune checkpoint blockade" *Science Translational Medicine*, Vol. vol. 12, 15 January 2020 (2020-01-15), article eaax7992, abstract, and page 1, right-hand column, last paragraph to page 2, left-hand column, paragraph 1 and page 9, right-hand column, paragraph 2 | 1-10, |
| Y | WEISS, J. M. et al. "Immunotherapy of cancer by IL-12-based cytokine combinations" *Expert Opin. Biol. Ther.*, Vol. 7, No. 11, 31 December 2007 (2007-12-31), page 1707 left-hand column, paragraph 2-page 1710, left-hand column, paragraph 1, and page 1711, right-hand column, paragraph 2-page-1713, left-hand column, paragraph 1 | 2-10 |
| A | WO 2021062406 A1 (ASKGENE PHARMA, INC.) 01 April 2021 (2021-04-01) entire document | 1-10 |
| A | WO 2020006274 A1 (INTREXON CORPORATION) 02 January 2020 (2020-01-02) entire document | 1-10 |

☑ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **11 July 2023** | **20 July 2023** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2023/092440**

**C.     DOCUMENTS CONSIDERED TO BE RELEVANT**

| Category* | Citation of document, with indication, where appropriate, of the relevant passages | Relevant to claim No. |
|-----------|-----------------------------------------------------------------------------------|-----------------------|
| A | US 2020405850 A1 (MASSACHUSETTS INSTITUTE OF TECHNOLOGY) 31 December 2020 (2020-12-31)<br>entire document | 1-10 |
| A | WO 2017201350 A1 (MODERNATX, INC.) 23 November 2017 (2017-11-23)<br>entire document | 1-10 |

Form PCT/ISA/210 (second sheet) (July 2022)

**INTERNATIONAL SEARCH REPORT**

| International application No. |
|---|
| **PCT/CN2023/092440** |

**Box No. I**      **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:

     a.   ☑   forming part of the international application as filed.

     b.   ☐   furnished subsequent to the international filing date for the purposes of international search (Rule 13*ter*.1(a)),

           ☐   accompanied by a statement to the effect that the sequence listing does not go beyond the disclosure in the international application as filed.

2.   ☐   With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

<div align="center">

**INTERNATIONAL SEARCH REPORT**
**Information on patent family members**

</div>

| International application No. |
| --- |
| **PCT/CN2023/092440** |

| Patent document cited in search report | | | Publication date (day/month/year) | Patent family member(s) | | | Publication date (day/month/year) |
| --- | --- | --- | --- | --- | --- | --- | --- |
| US | 2017136072 | A1 | 18 May 2017 | EP | 3293266 | A1 | 14 March 2018 |
| | | | | US | 10022405 | B2 | 17 July 2018 |
| | | | | US | 2016130317 | A1 | 12 May 2016 |
| | | | | US | 10258653 | B2 | 16 April 2019 |
| | | | | WO | 2008134879 | A1 | 13 November 2008 |
| | | | | US | 2010291043 | A1 | 18 November 2010 |
| | | | | US | 8765462 | B2 | 01 July 2014 |
| | | | | US | 2020038457 | A1 | 06 February 2020 |
| | | | | CA | 2723320 | A1 | 13 November 2008 |
| | | | | CA | 2723320 | C | 11 June 2019 |
| | | | | US | 2014370039 | A1 | 18 December 2014 |
| | | | | ES | 2654303 | T3 | 13 February 2018 |
| | | | | EP | 2150618 | A1 | 10 February 2010 |
| | | | | EP | 2150618 | A4 | 08 June 2011 |
| | | | | EP | 2150618 | B1 | 11 October 2017 |
| WO | 2021062406 | A1 | 01 April 2021 | EP | 4034551 | A1 | 03 August 2022 |
| | | | | JP | 2022549344 | A | 24 November 2022 |
| | | | | CA | 3153785 | A1 | 01 April 2021 |
| | | | | AU | 2020353235 | A1 | 31 March 2022 |
| | | | | US | 2022356221 | A1 | 10 November 2022 |
| WO | 2020006274 | A1 | 02 January 2020 | IL | 279277 | A | 31 January 2021 |
| | | | | AU | 2019291861 | A1 | 07 January 2021 |
| | | | | SG | 11202012203 | PA | 28 January 2021 |
| | | | | EP | 3813854 | A1 | 05 May 2021 |
| | | | | EP | 3813854 | A4 | 08 June 2022 |
| | | | | JP | 2021529199 | A | 28 October 2021 |
| | | | | CA | 3103372 | A1 | 02 January 2020 |
| | | | | US | 2021395773 | A1 | 23 December 2021 |
| US | 2020405850 | A1 | 31 December 2020 | WO | 2020263399 | A1 | 30 December 2020 |
| | | | | JP | 2022538974 | A | 07 September 2022 |
| | | | | US | 11642409 | B2 | 09 May 2023 |
| | | | | EP | 3990491 | A1 | 04 May 2022 |
| WO | 2017201350 | A1 | 23 November 2017 | JP | 2023072056 | A | 23 May 2023 |
| | | | | EP | 4186518 | A1 | 31 May 2023 |
| | | | | RU | 2018144292 | A | 18 June 2020 |
| | | | | RU | 2018144292 | A3 | 10 March 2021 |
| | | | | RU | 2769316 | C2 | 30 March 2022 |
| | | | | AU | 2017268397 | A1 | 17 January 2019 |
| | | | | PL | 3458083 | T3 | 13 March 2023 |
| | | | | JP | 2019516710 | A | 20 June 2019 |
| | | | | JP | 7246930 | B2 | 28 March 2023 |
| | | | | HRP | 20230050 | T1 | 03 March 2023 |
| | | | | MX | 2018013509 | A | 28 March 2019 |
| | | | | US | 2019125839 | A1 | 02 May 2019 |
| | | | | US | 10646549 | B2 | 12 May 2020 |
| | | | | US | 2021299221 | A1 | 30 September 2021 |
| | | | | US | 11311602 | B2 | 26 April 2022 |
| | | | | FI | 3458083 | T3 | 01 March 2023 |
| | | | | HUE | 061077 | T2 | 28 May 2023 |
| | | | | KR | 20220159479 | A | 02 December 2022 |

Form PCT/ISA/210 (patent family annex) (July 2022)

**INTERNATIONAL SEARCH REPORT**
Information on patent family members

International application No.

**PCT/CN2023/092440**

| Patent document cited in search report | Publication date (day/month/year) | Patent family member(s) | | | Publication date (day/month/year) |
|---|---|---|---|---|---|
| | | PT | 3458083 | T | 06 March 2023 |
| | | SI | 3458083 | T1 | 31 March 2023 |
| | | SG | 11201809381 | XA | 28 December 2018 |
| | | ES | 2941411 | T3 | 22 May 2023 |
| | | IL | 262501 | A | 31 December 2018 |
| | | EP | 3458083 | A1 | 27 March 2019 |
| | | EP | 3458083 | B1 | 02 November 2022 |
| | | LT | 3458083 | T | 10 February 2023 |
| | | CA | 3024470 | A1 | 23 November 2017 |
| | | US | 2020376081 | A1 | 03 December 2020 |
| | | US | 11000573 | B2 | 11 May 2021 |
| | | KR | 20190008890 | A | 25 January 2019 |
| | | KR | 102469450 | B1 | 22 November 2022 |
| | | DK | 3458083 | T3 | 30 January 2023 |
| | | RS | 63912 | B1 | 28 February 2023 |
| | | US | 2022265774 | A1 | 25 August 2022 |
| | | US | 11571463 | B2 | 07 February 2023 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 2022104869114
- CN 2023104938689

- US 7374930 B2